# EUROPÄISCHE PATENTSCHRIFT

(11) **EP 1 504 009 B1**
(45) Veröffentlichungstag und Bekanntmachung des Hinweises auf die Patenterteilung: **12.07.2006**
(21) Anmeldenummer: 03747437.6
(22) Anmeldetag: 30.04.2003
(51) Int. Cl.: C07D 487/04, A01N 43/90, C07C 69/612, C07C 69/65, C07C 69/738, C07D 249/00, C07D 239/00

(54) **FUNGIZIDE TRIAZOLOPYRIMIDINE, VERFAHREN ZU IHRER HERSTELLUNG UND IHRE VERWENDUNG ZUR BEK MPFUNG VON SCHADPILZEN SOWIE SIE ENT HALTENDE MITTEL**
FUNGICIDAL TRIAZOLOPYRIMIDINES, METHOD FOR THE PRODUCTION THEREOF, USE THEREOF FOR CONTROLLING HARMFUL FUNGI, AND AGENTS CONTAINING SAID FUNGICIDAL TRIAZOLOPYRIMIDINES
TRIAZOLOPYRIMIDINES FONGICIDES, LEURS PROCEDES DE PRODUCTION ET LEUR UTILISATION POUR LA LUTTE CONTRE DES CHAMPIGNONS NUISIBLES, ET AGENTS CONTENANT CES COMPOSES

(30) Priorität: 03.05.2002 DE 10219992
(43) Veröffentlichungstag der Anmeldung: 09.02.2005
(73) Patentinhaber: BASF Aktiengesellschaft, 67056 Ludwigshafen (DE)
(72) Erfinder: TORMO I BLASCO, Jordi, 69514 Laudenbach (DE); BLETTNER, Carsten, 68165 Mannheim (DE); MÜLLER, Bernd, 67227 Frankenthal (DE); GEWEHR, Markus, 56288 Kastellaun (DE); GRAMMENOS, Wassilios, 67071 Ludwigshafen (DE); GROTE, Thomas, 67157 Wachenheim (DE); GYPSER, Andreas, 68159 Mannheim (DE); RHEINHEIMER, Joachim, 67063 Ludwigshafen (DE); SCHÄFER, Peter, 67308 Ottersheim (DE); SCHIEWECK, Frank, 67258 Hessheim (DE); SCHWÖGLER, Anja, 68165 Mannheim (DE); AMMERMANN, Eberhard, 64646 Heppenheim (DE); STRATHMANN, Siegfried, 67117 Limburgerhof (DE); LORENZ, Gisela, 67434 Neustadt (DE); STIERL, Reinhard, 67251 Freinsheim (DE)
(86) Internationale Anmeldenummer: PCT/EP2003/004498
(87) Internationale Veröffentlichungsnummer: WO 2003/093271

(56) Entgegenhaltungen:
- WO-A-01/17972
- WO-A-96/35664
- FR-A- 2 765 875
- GB-A- 2 355 261
- US-A- 5 593 996

## Beschreibung

Die vorliegende Erfindung betrifft Triazolopyrimidine der Formel I, 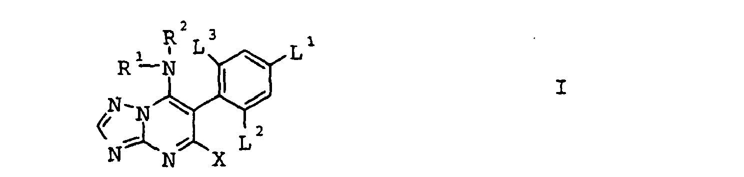 in die Substituenten folgende Bedeutung haben:
- L¹: C₁-C₄-Alkyl;
- L²: Halogen;
- L³: Wasserstoff oder Halogen;
- X: Halogen, Cyano, C₁-C₄-Alkyl, C₁-C₄-Alkoxy oder C₁-C₂-Halogenalkoxy;
- R¹,R²: unabhängig voneinander Wasserstoff, C₁-C₃-Alkyl, C₁-C₁₀-Halogenalkyl, C₃-C₈-Cycloalkyl, C₂-C₁₀-Alkenyl, C₄-C₁₀-Alkadienyl, C₂-C₁₀-Alkinyl oder C₄-C₁₀-Alkyl, welches kein Chiralitätszentrum aufweist, oder C₃-C₆-Cycloalkinyl, Phenyl, Naphthyl, oder ein fünf- bis zehngliedriger gesättigter, partiell ungesättigter oder aromatischer Heterocyclus, enthaltend ein bis vier Heteroatome aus der Gruppe O, N oder S,
R¹ und R² können auch zusammen mit dem Stickstoffatom, an das sie gebunden sind, einen fünf- oder sechsgliedrigen Ring bilden, der durch ein Atom aus der Gruppe O, N und S unterbrochen sein und/oder einen oder mehrere Substituenten aus der Gruppe Halogen, C₁-C₆-Alkyl, C₁-C₆-Halogenalkyl und Oxy-C₁-C₃-alkylenoxy tragen kann oder in dem ein N- und ein benachbartes C-Atom durch eine C₁-C₄-Alkylenkette verbunden sein können;
wobei R¹ und/oder R² durch eine bis drei gleiche oder verschiedene Gruppen R^{a} substituiert sein kann:
R^{a} Halogen, Cyano, Nitro, Hydroxy, C₁-C₆-Alkyl, C₁-C₆-Halogenalkyl, C₁-C₆-Alkylcarbonyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₂-C₆-Alkenyl, C₂-C₆-Alkenyloxy, C₃-C₆-Alkinyloxy, C₃-C₆-Cycloalkyl, Phenyl, Naphthyl, fünf- bis zehngliedriger gesättigter, partiell ungesättigter oder aromatischer Heterocyclus, enthaltend ein bis vier Heteroatome aus der Gruppe O, N oder S,
wobei diese aliphatischen, alicyclischen oder aromatischen Gruppen ihrerseits partiell oder vollständig halogeniert sein oder eine bis drei Gruppen R^{b} tragen können:
R^{b} Halogen, Cyano, Nitro, Hydroxy, Mercapto, Amino, Carboxyl, Aminocarbonyl, Aminothiocarbonyl, Alkyl, Haloalkyl, Alkenyl, Alkenyloxy, Alkinyloxy, Alkoxy, Halogenalkoxy, Alkylthio, Alkylamino, Dialkylamino, Formyl, Alkylcarbonyl, Alkylsulfonyl, Alkylsulfoxyl, Alkoxycarbonyl, Alkylcarbonyloxy, Alkylaminocarbonyl, Dialkylaminocarbonyl, Alkylaminothiocarbonyl, Dialkylaminothiocarbonyl, wobei die Alkylgruppen in diesen Resten 1 bis 6 Kohlenstoffatome enthalten und die genannten Alkenyl- oder Alkinylgruppen in diesen Resten 2 bis 8 Kohlenstoffatome enthalten;
und/oder einen bis drei der folgenden Reste:
Cycloalkyl, Cycloalkoxy, Heterocyclyl, Heterocyclyloxy, wobei die cyclischen Systeme 3 bis 10 Ringglieder enthalten; Aryl, Aryloxy, Arylthio, Aryl-C₁-C₆-alkoxy, Aryl-C₁-C₆-alkyl, Hetaryl, Hetaryloxy, Hetarylthio, wobei die Arylreste vorzugsweise 6 bis 10 Ringglieder, die Hetarylreste 5 oder 6 Ringglieder enthalten, wobei die cyclischen Systeme partiell oder vollständig halogeniert oder durch Alkyl- oder Haloalkylgruppen substituiert sein können.

Außerdem betrifft die Erfindung Verfahren und Zwischenprodukte zur Herstellung dieser Verbindungen, sie enthaltende Mittel sowie ihre Verwendung zur Bekämpfung von Schadpilzen.

Aus EP-A 71 792 und EP-A 550 113 sind einzelne 6-(4-Alkylphenyl)-triazolopyrimidine bekannt. In WO-A 98/46608 werden 5-Chlortriazolopyrimidine mit 7-Halogenalkylaminogruppen offenbart, dabei werden entsprechende 6-(4-Alkylphenyl)-triazolopyrimidine generisch umfasst. Die in den genannten Schriften beschriebenen Verbindungen sind zur Bekämpfung von Schadpilzen bekannt.

Ihre Wirkung ist jedoch in vielen Fällen nicht zufriedenstellend. Davon ausgehend, liegt der vorliegenden Erfindung die Aufgabe zugrunde, Verbindungen mit verbesserter Wirkung und/oder verbreitertem Wirkungsspektrum bereitzustellen.

Demgemäß wurden die eingangs definierten Verbindungen gefunden. Desweiteren wurden Verfahren und Zwischenprodukte zu ihrer Herstellung, sie enthaltende Mittel sowie Verfahren zur Bekämpfung von Schadpilzen unter Verwendung der Verbindungen I gefunden.

Die Verbindungen der Formel I unterscheiden sich von den aus den oben genannten Schriften in der Substitution der 6-(4-Alkylphenyl)gruppe, die zusätzlich in 2-Stellung ein Halogenatom trägt.

Die Verbindungen der Formel I weisen eine gegenüber den bekannten Verbindungen erhöhte Wirksamkeit gegen Schadpilze auf.

Die erfindungsgemäßen Verbindungen können auf verschiedenen Wegen erhalten werden. Vorteilhaft werden sie durch Umsetzung von 5-Aminotriazol der Formel II mit entsprechend substituierten Phenylmalonaten der Formel III, in der R für Alkyl, bevorzugt für C₁-C₆-Alkyl, insbesondere für Methyl oder Ethyl steht, dargestellt.

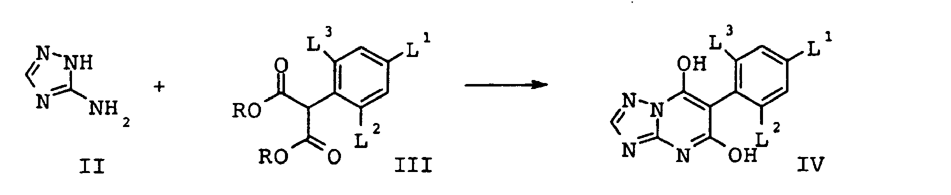

Diese Umsetzung erfolgt üblicherweise bei Temperaturen von 80°C bis 250°C, vorzugsweise 120°C bis 180°C, ohne Solvens oder in einem inerten organischen Lösungsmittel in Gegenwart einer Base [vgl. EP-A 770 615] oder in Gegenwart von Essigsäure unter den aus Adv. Het. Chem. Bd. 57, S. 81ff. (1993) bekannten Bedingungen.

Geeignete Lösungsmittel sind aliphatische Kohlenwasserstoffe, aromatische Kohlenwasserstoffe wie Toluol, o-, m- und p-Xylol, halogenierte Kohlenwasserstoffe, Ether, Nitrile, Ketone, Alkohole, sowie N-Methylpyrrolidon, Dimethylsulfoxid, Dimethylformamid und Dimethylacetamid. Besonders bevorzugt wird die Umsetzung ohne Lösungsmittel oder in Chlorbenzol, Xylol, Dimethylsulfoxid, N-Methylpyrrolidon durchgeführt. Es können auch Gemische der genannten Lösungsmittel verwendet werden.

Als Basen kommen allgemein anorganische Verbindungen wie Alkalimetall- und Erdalkalimetallhydroxide, Alkalimetall- und Erdalkalimetalloxide, Alkalimetall- und Erdalkalimetallhydride, Alkalimetallamide, Alkalimetall- und Erdalkalimetallcarbonate sowie Alkalimetallhydrogencarbonate, metallorganische Verbindungen, insbesondere Alkalimetallalkyle, Alkylmagnesiumhalogenide sowie Alkalimetall- und Erdalkalimetallalkoholate und Dimethoxymagnesium, außerdem organische Basen, z.B. tertiäre Amine wie Trimethylamin, Triethylamin, Tri-isopropylethylamin, Tributylamin und N-Methylpiperidin, N-Methylmorpholin, Pyridin, substituierte Pyridine wie Collidin, Lutidin und 4-Dimethylaminopyridin sowie bicyclische Amine in Betracht. Besonders bevorzugt werden tertiäre amine wie Tri-isopropylethylamin, Tributylamin, N-Methylmorpholin oder N-Methylpiperidin.

Die Basen werden im allgemeinen in katalytischen Mengen eingesetzt, sie können aber auch äquimolar, im Überschuß oder gegebenenfalls als Lösungsmittel verwendet werden.

Die Edukte werden im allgemeinen in äquimolaren Mengen miteinander umgesetzt. Es kann für die Ausbeute vorteilhaft sein, die Base und das Malonat III in einem Überschuß bezogen auf das Triazol einzusetzen.

Phenylmalonate der Formel III werden vorteilhaft aus der Reaktion entsprechend substituierter Brombenzole mit Dialkylmalonaten unter Cu(I)-Katalyse erhalten [vgl. Chemistry Letters, S. 367-370, 1981; EP-A 10 02 788].

Die Dihydroxytriazolopyrimidine der Formel IV werden unter den aus WO-A 94/20501 bekannten Bedingungen in die Dihalogenpyrimidine der Formel V überführt. Als Halogenierungsmittel wird vorteilhaft ein Chlorierungsmittel oder ein Bromierungsmittel, wie Phosphoroxybromid oder Phosphoroxychlorid, ggf. in Anwesenheit eines Lösungsmittels, eingesetzt.

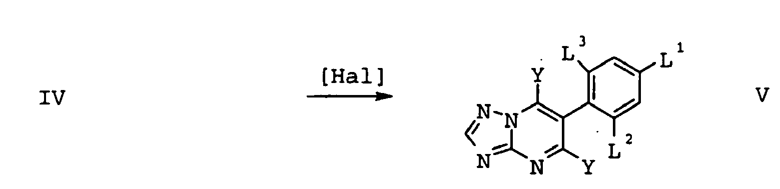

Diese Umsetzung wird üblicherweise bei 0°C bis 150°C, bevorzugt bei 80°C bis 125°C, durchgeführt [vgl. EP-A 770 615].

Dihalogenpyrimidine der Formel V werden mit Aminen der Formel VI, 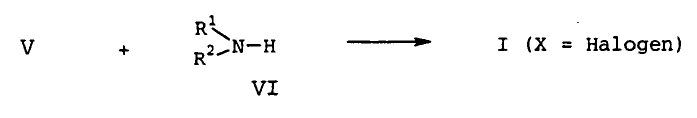 in der R¹ und R² wie in Formel I definiert sind, zu Verbindungen der Formel I, in der X für Halogen steht, weiter umgesetzt.

Diese Umsetzung wird vorteilhaft bei 0°C bis 70°C, bevorzugt 10°C bis 35°C durchgeführt, vorzugsweise in Anwesenheit eines inerten Lösungsmittels, wie Ether, z. B. Dioxan, Diethylether oder insbesondere Tetrahydrofuran, halogenierte Kohlenwasserstoffe, wie Dichloromethan und aromatische Kohlenwasserstoffe, wie beispielsweise Toluol [vgl. WO-A 98/46608].

Die Verwendung einer Base, wie tertiäre Amine, beispielsweise Triethylamin oder anorganische Amine, wie Kaliumcarbonat ist bevorzugt; auch überschüssiges Amin der Formel VI kann als Base dienen.

Verbindungen der Formel I, in der X Cyano, C₁-C₆-Alkoxy oder C₁-C₂-Halogenalkoxy bedeutet, können vorteilhaft aus der Umsetzung von Verbindungen I, in der X Halogen, bevorzugt Chlor bedeutet, mit Verbindungen M-X' (Formel VII) erhalten werden. Verbindungen VII stellen je nach der Bedeutung der einzuführenden Gruppe X' ein anorganisches Cyanid, ein Alkoxylat oder ein Halogenalkoxylat dar. Die Umsetzung erfolgt vorteilhaft in Anwesenheit eines inerten Lösungsmittels. Das Kation M in Formel VII hat geringe Bedeutung; aus praktischen Gründen sind üblicherweise Ammonium-, Tetraalkylammonium- oder Alkali- oder Erdalkalimetallsalze bevorzugt.

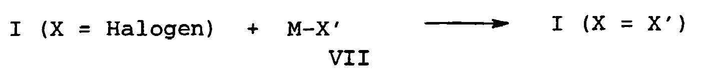

Üblicherweise liegt die Reaktionstemperatur bei 0 bis 120°C, bevorzugt bei 10 bis 40°C [vgl. J. Heterocycl. Chem., Bd.12, S. 861-863 (1975)].

Geeignete Lösungsmittel umfassen Ether, wie Dioxan, Diethylether und, bevorzugt Tetrahydrofuran, halogenierte Kohlenwasserstoffe wie Dichloromethan und aromatische Kohlenwasserstoffe, wie Toluol.

Verbindungen der Formel I, in denen X für C₁-C₄-Alkyl steht, können vorteilhaft durch folgenden Syntheseweg erhalten werden:

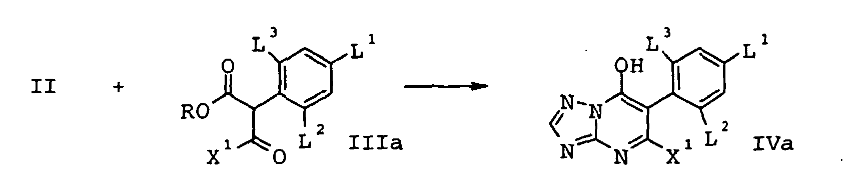

Ausgehend von den Diketonen IIIa werden die 5-Alkyl-7-hydroxy-6-phenyltriazolopyrimidine IVa erhalten. Durch Verwendung der leicht zugänglichen 2-Phenylacetessigestern (IIIa mit X¹=CH₃) werden die 5-Methyl-7-hydroxy-6-phenyltriazolopyrimidine erhalten [vgl. Chem. Pharm. Bull., 9, 801, (1961)]. Die Herstellung der Ausgangsverbindungen IIIa erfolgt vorteilhaft unter den aus EP-A 10 02 788 beschrieben Bedingungen.

Die so erhaltenen 5-Alkyl-7-hydroxy-6-phenyltriazolopyrimidine werden mit Halogenierungsmitteln zu den 7-Halogenotriazolopyrimidinen der Formel Va umgesetzt. Bevorzugt werden Chlorierungs- oder Bromierungsmittel wie Phosphoroxybromid, Phosphoroxychlorid, Thionylchlorid, Thionylbromid oder Sulfurylchlorid eingesetzt. Die Umsetzung kann in Substanz oder in Gegenwart eines Lösungsmittels durchgeführt werden. Übliche Reaktionstemperaturen betragen von 0 bis 150°C oder vorzugsweise von 80 bis 125°C.

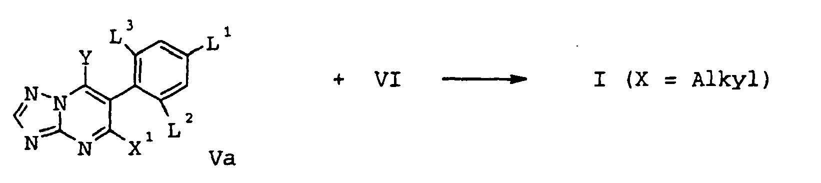

Die Umsetzung von Va mit Aminen VI erfolgt unter den weiter oben beschriebenen Bedingungen.

Verbindungen der Formel I in der X C₁-C₄-Alkyl bedeutet, können alternativ auch aus Verbindungen I, in der X Halogen, insbesondere Chlor, bedeutet und Malonaten der Formel VIII hergestellt werden. In Formel VIII bedeuten X" Wasserstoff oder C₁-C₃-Alkyl und R C₁-C₄-Alkyl. Sie werden zu Verbindungen der Formel IX umgesetzt und zu Verbindungen I decarboxyliert [vgl. US 5,994,360].

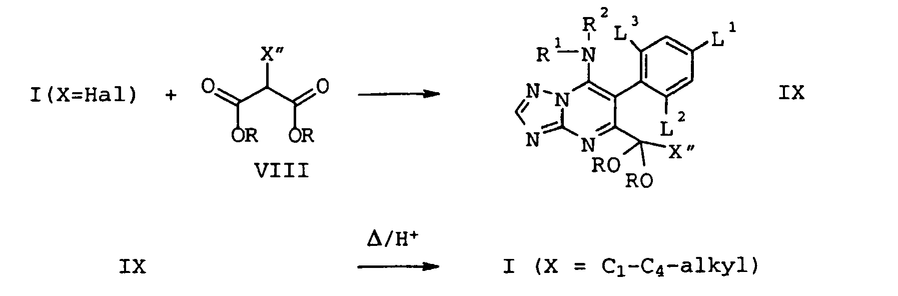

Die Malonate VIII sind in der Literatur bekannt [J. Am. Chem. Soc., Bd. 64, 2714 (1942); J. Org. Chem., Bd. 39, 2172 (1974); Helv. Chim. Acta, Bd. 61, 1565 (1978)] oder können gemäß der zitierten Literatur hergestellt werden.

Die anschließende Verseifung des Esters IX erfolgt unter allgemein üblichen Bedingungen, in Abhängigkeit der verschiedenen Strukturelemente kann die alkalische oder die saure Verseifung der Verbindungen IX vorteilhaft sein. Unter den Bedingungen der Esterverseifung kann die Decarboxylierung zu I bereits ganz oder teilweise erfolgen.

Die Decarboxylierung erfolgt üblicherweise bei Temperaturen von 20°C bis 180°C, vorzugsweise 50°C bis 120°C, in einem inerten Lösungsmittel, gegebenenfalls in Gegenwart einer Säure.

Geeignete Säuren sind Salzsäure, Schwefelsäure, Phosphorsäure, Ameisensäure, Essigsäure, p-Toluolsulfonsäure. Geeignete Lösungsmittel sind Wasser, aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol, sowie Dimethylsulfoxid, Dimethylformamid und Dimethylacetamid, besonders bevorzugt wird die Reaktion in Salzsäure oder Essigsäure durchgeführt. Es können auch Gemische der genannten Lösungsmittel verwendet werden.

Verbindungen der Formel I, in denen X für C₁-C₄-Alkyl steht, können auch durch Kupplung von 5-Halogentriazolopyrimidinen der Formel I, in der X Halogen bedeutet, mit metallorganischen Reagenzien der Formel X erhalten werden. In einer Ausführungsform dieses Verfahrens erfolgt die Umsetzung unter Übergangsmetallkatalyse, wie Ni- oder Pd-Katalyse.

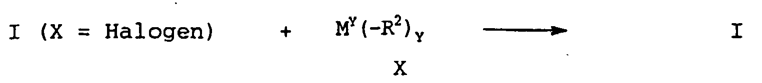

In Formel X steht M für ein Metallion der Wertigkeit Y, wie beispielsweise B, Zn oder Sn. Diese Reaktion kann beispielsweise analog folgender Methoden durchgeführt werden: J. Chem. Soc. Perkin Trans. 1, 1187 (1994), ebenda 1, 2345 (1996); WO-A 99/41255; Aust. J. Chem., Bd. 43, 733 (1990); J. Org. Chem., Bd. 43, 358 (1978); J. Chem. Soc. Chem. Commun. 866 (1979); Tetrahedron Lett., Bd. 34, 8267 (1993); ebenda, Bd. 33, 413 (1992).

Sofern R¹ oder R² Halogenalkyl oder Halogenalkenylgruppen beinhalten ist für optisch aktive Amine der Formel VI die (S)-Konfiguration bevorzugt.

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischen- und Endprodukte fallen z.T. in Form farbloser oder schwach bräunlicher, zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.

Sofern einzelne Verbindungen I nicht auf den voranstehend beschriebenen Wegen zugänglich sind, können sie durch Derivatisierung anderer Verbindungen I hergestellt werden.

Sofern bei der Synthese Isomerengemische anfallen, ist im allgemeinen jedoch eine Trennung nicht unbedingt erforderlich, da sich die einzelnen Isomere teilweise während der Aufbereitung für die Anwendung oder bei der Anwendung (z.B. unter Licht-, Säure- oder Baseneinwirkung) ineinander umwandeln können. Entsprechende Umwandlungen können auch nach der Anwendung, beispielsweise bei der Behandlung von Pflanzen in der behandelten Pflanze oder im zu bekämpfenden Schadpilz erfolgen.

Bei den in den vorstehenden Formeln angegebenen Definitionen der Symbole wurden Sammelbegriffe verwendet, die allgemein repräsentativ für die folgenden Substituenten stehen:
**Halogen:** Fluor, Chlor, Brom und Jod;
**Alkyl**: gesättigte, geradkettige oder verzweigte Kohlenwasserstoffreste mit 1 bis 4, 6, 8 oder 10 Kohlenstoffatomen, z.B. C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;
Halogenalkyl: geradkettige oder verzweigte Alkylgruppen mit 1 bis 10 Kohlenstoffatomen (wie vorstehend genannt), wobei in diesen Gruppen teilweise oder vollständig die Wasserstoffatome durch Halogenatome wie vorstehend genannt ersetzt sein können, z.B. C₁-C₂-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl oder 1,1,1-Trifluorprop-2-yl;
**Alkenyl:** ungesättigte, geradkettige oder verzweigte Kohlenwasserstoffreste mit 2 bis 4, 6, 8 oder 10 Kohlenstoffatomen und einer Doppelbindung in einer beliebigen Position, z.B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-lpropenyl und 1-Ethyl-2-methyl-2-propenyl;
**Alkadienyl:** ungesättigte, geradkettige oder verzweigte Kohlenwasserstoffreste mit 4, 6, 8 oder 10 Kohlenstoffatomen und zwei Doppelbindungen in beliebiger Position;
Halogenalkenyl: ungesättigte, geradkettige oder verzweigte Kohlenwasserstoffreste mit 2 bis 10 Kohlenstoffatomen und einer Doppelbindung in einer beliebigen Position (wie vorstehend genannt), wobei in diesen Gruppen die Wasserstoffatome teilweise oder vollständig gegen Halogenatome wie vorstehend genannt, insbesondere Fluor, Chlor und Brom, ersetzt sein können;
**Alkinyl:** geradkettige oder verzweigte Kohlenwasserstoffgruppen mit 2 bis 4, 6, 8 oder 10 Kohlenstoffatomen und einer Dreifachbindung in einer beliebigen Position, z.B. C₂-C₆-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl;
**Halogenalkinyl:** ungesättigte, geradkettige oder verzweigte Kohlenwasserstoffreste mit 2 bis 8 Kohlenstoffatomen und einer Dreifachbindung in einer beliebigen Position (wie vorstehend genannt), wobei in diesen Gruppen die Wasserstoffatome teilweise oder vollständig gegen Halogenatome wie vorstehend genannt, insbesondere Fluor, Chlor und Brom, ersetzt sein können;
Cycloalkyl: mono- oder bicyclische, gesättigte Kohlenwasserstoffgruppen mit 3 bis 6 oder 8 Kohlenstoffringgliedern, z.B. C₃-C₈-Cycloalkyl wie Cyclopropyl, Cyclobutyl, Cyclopentyl, Cyclohexyl, Cycloheptyl und Cyclooctyl;
**Oxyalkylenoxy:** divalente unverzweigte Ketten aus 1 bis 3 CH₂-Gruppen, wobei beide Valenzen über ein Sauerstoffatom an das Gerüst gebunden ist, z.B. OCH₂O, OCH₂CH₂O und OCH₂CH₂CH₂O;

**fünf- bis zehngliedriger gesättigter, partiell ungesättigter oder aromatischer Heterocyclus, enthaltend ein bis vier Heteroatome aus der Gruppe O, N oder S:**
- **5- oder 6-gliedriges Heterocyclyl**, enthaltend ein bis drei Stickstoffatome und/oder ein Sauerstoff- oder Schwefelatom oder ein oder zwei Sauerstoff- und/oder Schwefelatome, z.B. 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 2-Pyrrolin-2-yl, 2-Pyrrolin-3-yl, 3-Pyrrolin-2-yl, 3-Pyrrolin-3-yl, 2-Piperidinyl, 3-Piperidinyl, 4-Piperidinyl, 1,3-Dioxan-5-yl, 2-Tetrahydropyranyl, 4-Tetrahydropyranyl, 2-Tetrahydrothienyl, 3-Hexahydropyridazinyl, 4-Hexahydropyridazinyl, 2-Hexahydropyrimidinyl, 4-Hexahydropyrimidinyl, 5-Hexahydropyrimidinyl und 2-Piperazinyl;
- 5-gliedriges Heteroaryl, enthaltend ein bis vier Stickstoffatome oder ein bis drei Stickstoffatome und ein Schwefel- oder Sauerstoffatom: 5-Ring Heteroarylgruppen, welche neben Kohlenstoffatomen ein bis vier Stickstoffatome oder ein bis drei Stickstoffatome und ein Schwefel- oder Sauerstoffatom als Ringglieder enthalten können, z.B. 2-Furyl, 3-Furyl, 2-Thienyl, 3-Thienyl, 2-Pyrrolyl, 3-Pyrrolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, und 1,3,4-Triazol-2-yl;
- **6-gliedriges Heteroaryl,** enthaltend ein bis drei bzw. ein bis vier Stickstoffatome: 6-Ring Heteroarylgruppen, welche neben Kohlenstoffatomen ein bis drei bzw. ein bis vier Stickstoffatome als Ringglieder enthalten können, z.B. 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl und 2-Pyrazinyl; **Alkylen:** divalente unverzweigte Ketten aus 3 bis 5 CH₂-Gruppen, z.B. CH₂, CH₂CH₂, CH₂CH₂CH₂, CH₂CH₂CH₂CH₂ und CH₂CH₂CH₂CH₂CH₂;
   **Oxyalkylen:** divalente unverzweigte Ketten aus 2 bis 4 CH₂-Gruppen, wobei eine Valenz über ein Sauerstoffatom an das Gerüst gebunden ist, z.B. OCH₂CH₂, OCH₂CH₂CH₂ und OCH₂CH₂CH₂CH₂;
   **Oxyalkylenoxy:** divalente unverzweigte Ketten aus 1 bis 3 CH₂-Gruppen, wobei beide Valenzen über ein Sauerstoffatom an das Gerüst gebunden ist, z.B. OCH₂O, OCH₂CH₂O und OCH₂CH₂CH₂O.

In dem Umfang der vorliegenden Erfindung sind die (R)- und (S)-Isomere und die Razemate von Verbindungen der Formel I eingeschlossen, die chirale Zentren aufweisen.

Die besonders bevorzugten Ausführungsformen der Zwischenprodukte in Bezug auf die Variablen entsprechen denen der Reste L¹, L², L³, R¹, R² und X der Formel I.

Im Hinblick auf ihre bestimmungsgemäße Verwendung der Triazolopyrimidine der Formel I sind die folgenden Bedeutungen der Substituenten, und zwar jeweils für sich allein oder in Kombination, besonders bevorzugt:

Verbindungen I werden bevorzugt, in denen R¹ für C₁-C₃-Alkyl oder C₁-C₈-Halogenalkyl steht.

Gleichermaßen bevorzugt sind Verbindungen I, in denen R¹ für einen 5- oder 6-gliedrigen gesättigten oder aromatischen Heterocyclus steht.

Darüber hinaus sind Verbindungen I bevorzugt, in denen R¹ C₂-C₁₀-Alkenyl bedeutet.

Verbindungen I sind besonders bevorzugt, in denen R¹ für eine Gruppe B steht 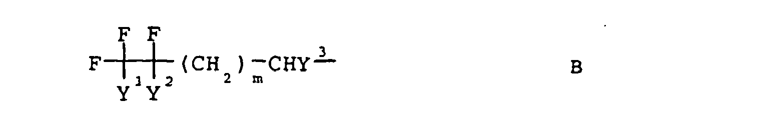 worin
- Y¹: Wasserstoff, Fluor oder C₁-C₆-Fluoroalkyl,
- Y²: Wasserstoff oder Fluor, oder Y¹ und Y² bilden gemeinsam eine Doppelbindung;
- m: is 0 oder 1; und
- Y³: Wasserstoff oder Methyl bedeuten.

Außerdem werden Verbindungen I bevorzugt, in denen R¹ für C₃-C₆-Cycloalkyl steht, welches durch C₁-C₄-Alkyl substituiert sein kann.

Insbesondere werden Verbindungen I bevorzugt, in denen R² Wasserstoff bedeutet.

Gleichermaßen bevorzugt sind Verbindungen I, in denen R² für Methyl oder Ethyl steht.

Sofern R¹ und/oder R² Halogenalkyl oder Halogenalkenylgruppen mit Chiralitätszentrum beinhalten, sind die (S)-Isomere bevorzugt.

Weiterhin werden Verbindungen I besonders bevorzugt, in denen R¹ und R² zusammen mit dem Stickstoffatom, an das sie gebunden sind, einen fünf- oder sechsgliedrigen Ring bilden, der durch ein Atom aus der Gruppe O, N und S unterbrochen sein und/oder einen oder mehrere Substituenten aus der Gruppe Halogen, C₁-C₆-Alkyl, C₁-C₆-Halogenalkyl und Oxy-C₁-C₃-alkylenoxy tragen kann oder in dem ein N- und ein benachbartes C-Atom durch eine C₁-C₄-Alkylenkette verbunden sein können.

Insbesondere werden Verbindungen I besonders bevorzugt, in denen R¹ und R² zusammen mit dem Stickstoffatom, an das sie gebunden sind, einen Piperidinylring bilden, der ggf. durch eine bis drei Gruppen Halogen, C₁-C₄-Alkyl oder C₁-C₄-Halogenalkyl, insbesondere durch 4-Methyl substituiert ist.

Außerdem werden Verbindungen I besonders bevorzugt, in denen L¹ Methyl oder Ethyl, insbesondere Methyl bedeutet.

Gleichermaßen besonders bevorzugt sind Verbindungen I, in denen L³ Halogen bedeutet.

Verbindungen I werden besonders bevorzugt, in denen X Halogen oder C₁-C₄-Alkyl, wie Chlor oder Methyl, insbesondere Chlor bedeutet.

Insbesondere sind im Hinblick auf ihre Verwendung die in den folgenden Tabellen zusammengestellten Verbindungen I bevorzugt. Die in den Tabellen für einen Substituenten genannten Gruppen stellen außerdem für sich betrachtet, unabhängig von der Kombination, in der sie genannt sind, eine besonders bevorzugte Ausgestaltung des betreffenden Substituenten dar.

### Tabelle 1

Verbindungen der Formel I, in denen X für Chlor, L¹ für Methyl, L² für Fluor und L³ für Wasserstoff stehen und die Kombination von R¹ und R² für eine Verbindung jeweils einer Zeile der Tabelle A entspricht

### Tabelle 2

Verbindungen der Formel I, in denen X für Chlor, L¹ für Methyl, L² und L³ für Fluor stehen und die Kombination von R¹ und R² für eine Verbindung jeweils einer Zeile der Tabelle A entspricht

### Tabelle 3

Verbindungen der Formel I, in denen X für Chlor, L¹ für Methyl, L² für Chlor und L³ für Wasserstoff stehen und die Kombination von R¹ und R² für eine Verbindung jeweils einer Zeile der Tabelle A entspricht

### Tabelle 4

Verbindungen der Formel I, in denen X für Chlor, L¹ für Methyl, L² und L³ für Chlor stehen und die Kombination von R¹ und R² für eine Verbindung jeweils einer Zeile der Tabelle A entspricht

### Tabelle 5

Verbindungen der Formel I, in denen X für Chlor, L¹ für Ethyl, L² für Fluor und L³ für Wasserstoff stehen und die Kombination von R¹ und R² für eine Verbindung jeweils einer Zeile der Tabelle A entspricht

### Tabelle 6

Verbindungen der Formel I, in denen X für Chlor, L¹ für Ethyl, L² und L³ für Fluor stehen und die Kombination von R¹ und R² für eine Verbindung jeweils einer Zeile der Tabelle A entspricht

### Tabelle 7

Verbindungen der Formel I, in denen X für Chlor, L¹ für Ethyl, L² für Chlor und L³ für Wasserstoff stehen und die Kombination von R¹ und R² für eine Verbindung jeweils einer Zeile der Tabelle A entspricht

### Tabelle 8

Verbindungen der Formel I, in denen X für Chlor, L¹ für Ethyl, L² und L³ für Chlor stehen und die Kombination von R¹ und R² für eine Verbindung jeweils einer Zeile der Tabelle A entspricht

### Tabelle 9

Verbindungen der Formel I, in denen X für Cyano, L¹ für Methyl, L² für Fluor und L³ für Wasserstoff stehen und die Kombination von R¹ und R² für eine Verbindung jeweils einer Zeile der Tabelle A entspricht

### Tabelle 10

Verbindungen der Formel I, in denen X für Cyano, L¹ für Methyl, L² und L³ für Fluor stehen und die Kombination von R¹ und R² für eine Verbindung jeweils einer Zeile der Tabelle A entspricht

### Tabelle 11

Verbindungen der Formel I, in denen X für Cyano, L¹ für Methyl, L² für Chlor und L³ für Wasserstoff stehen und die Kombination von R¹ und R² für eine Verbindung jeweils einer Zeile der Tabelle A entspricht

### Tabelle 12

Verbindungen der Formel I, in denen X für Cyano, L¹ für Methyl, L² und L³ für Chlor stehen und die Kombination von R¹ und R² für eine Verbindung jeweils einer Zeile der Tabelle A entspricht

### Tabelle 13

Verbindungen der Formel I, in denen X für Cyano, L¹ für Ethyl, L² für Fluor und L³ für Wasserstoff stehen und die Kombination von R¹ und R² für eine Verbindung jeweils einer Zeile der Tabelle A entspricht

### Tabelle 14

Verbindungen der Formel I, in denen X für Cyano, L¹ für Ethyl, L² und L³ für Fluor stehen und die Kombination von R¹ und R² für eine Verbindung jeweils einer Zeile der Tabelle A entspricht

### Tabelle 15

Verbindungen der Formel I, in denen X für Cyano, L¹ für Ethyl, L² für Chlor und L³ für Wasserstoff stehen und die Kombination von R¹ und R² für eine Verbindung jeweils einer Zeile der Tabelle A entspricht

### Tabelle 16

Verbindungen der Formel I, in denen X für Cyano, L¹ für Ethyl, L² und L³ für Chlor stehen und die Kombination von R¹ und R² für eine Verbindung jeweils einer Zeile der Tabelle A entspricht

### Tabelle 17

Verbindungen der Formel I, in denen X und L¹ für Methyl, L² für Fluor und L³ für Wasserstoff stehen und die Kombination von R¹ und R² für eine Verbindung jeweils einer Zeile der Tabelle A entspricht

### Tabelle 18

Verbindungen der Formel I, in denen X und L¹ für Methyl, L² und L³ für Fluor stehen und die Kombination von R¹ und R² für eine Verbindung jeweils einer Zeile der Tabelle A entspricht

### Tabelle 19

Verbindungen der Formel I, in denen X und L¹ für Methyl, L² für Chlor und L³ für Wasserstoff stehen und die Kombination von R¹ und R² für eine Verbindung jeweils einer Zeile der Tabelle A entspricht

### Tabelle 20

Verbindungen der Formel I, in denen X und L¹ für Methyl, L² und L³ für Chlor stehen und die Kombination von R¹ und R² für eine Verbindung jeweils einer Zeile der Tabelle A entspricht

### Tabelle 21

Verbindungen der Formel I, in denen X für Methyl, L¹ für Ethyl, L² für Fluor und L³ für Wasserstoff stehen und die Kombination von R¹ und R² für eine Verbindung jeweils einer Zeile der Tabelle A entspricht

### Tabelle 22

Verbindungen der Formel I, in denen X für Methyl, L¹ für Ethyl, L² und L³ für Fluor stehen und die Kombination von R¹ und R² für eine Verbindung jeweils einer Zeile der Tabelle A entspricht

### Tabelle 23

Verbindungen der Formel I, in denen X für Methyl, L¹ für Ethyl, L² für Chlor und L³ für Wasserstoff stehen und die Kombination von R¹ und R² für eine Verbindung jeweils einer Zeile der Tabelle A entspricht

### Tabelle 24

Verbindungen der Formel I, in denen X für Methyl, L¹ für Ethyl, L² und L³ für Chlor stehen und die Kombination von R¹ und R² für eine Verbindung jeweils einer Zeile der Tabelle A entspricht

### Tabelle 25

Verbindungen der Formel I, in denen X für Methoxy, L¹ für Methyl, L² für Fluor und L³ für Wasserstoff stehen und die Kombination von R¹ und R² für eine Verbindung jeweils einer Zeile der Tabelle A entspricht

### Tabelle 26

Verbindungen der Formel I, in denen X für Methoxy, L¹ für Methyl, L² und L³ für Fluor stehen und die Kombination von R¹ und R² für eine Verbindung jeweils einer Zeile der Tabelle A entspricht

### Tabelle 27

Verbindungen der Formel I, in denen X für Methoxy, L¹ für Methyl, L² für Chlor und L³ für Wasserstoff stehen und die Kombination von R¹ und R² für eine Verbindung jeweils einer Zeile der Tabelle A entspricht

### Tabelle 28

Verbindungen der Formel I, in denen X für Methoxy, L¹ für Methyl, L² und L³ für Chlor stehen und die Kombination von R¹ und R² für eine Verbindung jeweils einer Zeile der Tabelle A entspricht

### Tabelle 29

Verbindungen der Formel I, in denen X für Methoxy, L¹ für Ethyl, L² für Fluor und L³ für Wasserstoff stehen und die Kombination von R¹ und R² für eine Verbindung jeweils einer Zeile der Tabelle A entspricht

### Tabelle 30

Verbindungen der Formel I, in denen X für Methoxy, L¹ für Ethyl, L² und L³ für Fluor stehen und die Kombination von R¹ und R² für eine Verbindung jeweils einer Zeile der Tabelle A entspricht

### Tabelle 31

Verbindungen der Formel I, in denen X für Methoxy, L¹ für Ethyl, L² für Chlor und L³ für Wasserstoff stehen und die Kombination von R¹ und R² für eine Verbindung jeweils einer Zeile der Tabelle A entspricht

### Tabelle 32

Verbindungen der Formel I, in denen X für Methoxy, L¹ für Ethyl, L² und L³ für Chlor stehen und die Kombination von R¹ und R² für eine Verbindung jeweils einer Zeile der Tabelle A entspricht

**Tabelle A**

| **No.** | R¹ | R² |
|---|---|---|
| A-1 | H | H |
| A-2 | CH₂CH₃ | H |
| A-3 | CH₂CH₃ | CH₃ |
| A-4 | CH₂CH₃ | CH₂CH₃ |
| A-5 | CH₂CF₃ | H |
| A-6 | CH₂CF₃ | CH₃ |
| A-7 | CH₂CF₃ | CH₂CH₃ |
| A-8 | CH₂CCl₃ | H |
| A-9 | CH₂CCl₃ | CH₃ |
| A-10 | CH₂CCl₃ | CH₂CH₃ |
| A-11 | CH₂CH₂CH₃ | H |
| A-12 | CH₂CH₂CH₃ | CH₃ |
| A-13 | CH₂CH₂CH₃ | CH₂CH₃ |
| A-14 | CH₂CH₂CH₃ | CH₂CH₂CH₃ |
| A-15 | CH (CH₃)₂ | H |
| A-16 | CH(CH₃)₂ | CH₃ |
| A-17 | CH(CH₃)₂ | CH₂CH₃ |
| A-18 | (±) CH(CH₃)-CF₃ | H |
| A-19 | (±) CH(CH₃)-CF₃ | CH₃ |
| A-20 | (±) CH(CH₃)-CF₃ | CH₂CH₃ |
| A-21 | (S) CH(CH₃)-CF₃ | H |
| A-22 | (S) CH(CH₃)-CF₃ | CH₃ |
| A-23 | (S) CH(CH₃)-CF₃ | CH₂CH₃ |
| A-24 | (R) CH (CH₃) -CF₃ | H |
| A-25 | (R) CH(CH₃)-CF₃ | CH₃ |
| A-26 | (R) CH(CH₃)-CF₃ | CH₂CH₃ |
| A-27 | (±) CH(CH₃)-CCl₃ | H |
| A-28 | (±) CH(CH₃)-CCl₃ | CH₃ |
| A-29 | (±) CH(CH₃)-CCl₃ | CH₂CH₃ |
| A-30 | (S) CH(CH₃)-CCl₃ | H |
| A-31 | (S) CH(CH₃)-CCl₃ | CH₃ |
| A-32 | (S) CH (CH₃) -CCl₃ | CH₂CH₃ |
| A-33 | (R) CH(CH₃)-CCl₃ | H |
| A-34 | (R) CH(CH₃)-CCl₃ | CH₃ |
| A-35 | (R) CH(CH₃)-CCl₃ | CH₂CH₃ |
| A-36 | CH₂CF₂CF₃ | H |
| A-37 | CH₂CF₂CF₃ | CH₃ |
| A-38 | CH₂CF₂CF₃ | CH₂CH₃ |
| A-39 | CH₂(CF₂)₂CF₃ | H |
| A-40 | CH₂(CF₂)₂CF₃ | CH₃ |
| A-41 | CH₂(CF₂)₂CF₃ | CH₂CH₃ |
| A-42 | CH₂C(CH₃)=CH₂ | H |
| A-43 | CH₂C(CH₃)=CH₂ | CH₃ |
| A-44 | CH₂C(CH₃)=CH₂ | CH₂CH₃ |
| A-45 | cyclopentyl | H |
| A-46 | cyclopentyl | CH₃ |
| A-47 | cyclopentyl | CH₂CH₃ |
| A-48 | Cyclohexyl | H |
| A-49 | Cyclohexyl | CH₃ |
| A-50 | Cyclohexyl | CH₂CH₃ |
| A-51 | -(CH₂)₂CH=CHCH₂- | |
| A-52 | -(CH₂)₂C(CH₃)=CHCH₂- | |
| A-53 | -(CH₂)₂CH(CH₃)(CH₂)₂- | |
| A-54 | -(CH₂)₂CHF(CH₂)₂- | |
| A-55 | -(CH₂)₃CHFCH₂- | |
| A-56 | -(CH₂)₂CH(CF₃)(CH₂)₂- | |
| A-57 | -(CH₂)₂O(CH₂)₂- | |
| A-58 | -(CH₂)₂S(CH₂)₂- | |
| A-59 | -(CH₂)₅- | |
| A-60 | -(CH₂)₄- | |
| A-61 | -CH₂CH=CHCH₂- | |
| A-62 | -CH(CH₃)(CH₂)₃- | |
| A-63 | -CH₂CH(CH₃)(CH₂)₂- | |

Die Verbindungen I eignen sich als Fungizide. Sie zeichnen sich durch eine hervorragende Wirksamkeit gegen ein breites Spektrum von pflanzenpathogenen Pilzen, insbesondere aus der Klasse der *Ascomyceten, Deuteromyceten, Phycomyceten* und *Basidiomyceten,* aus. Sie sind zum Teil systemisch wirksam und können im Pflanzenschutz als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Bananen, Baumwolle, Soja, Kaffee, Zuckerrohr, Wein, Obst- und Zierpflanzen und Gemüsepflanzen wie Gurken, Bohnen, Tomaten, Kartoffeln und Kürbisgewächsen, sowie an den Samen dieser Pflanzen.

Speziell eignen sie sich zur Bekämpfung folgender Pflanzenkrankheiten:
- Alternaria-Arten an Gemüse und Obst,
- Botrytis cinerea (Grauschimmel) an Erdbeeren, Gemüse, Zierpflanzen und Reben,
- Cercospora arachidicola an Erdnüssen,
- Erysiphe cichoracearum und Sphaerotheca fuliginea an Kürbisgewächsen,
- *Blumeria graminis* (echter Mehltau) an Getreide,
- *Fusarium-* und *Verticillium-*Arten an verschiedenen Pflanzen,
- *Helminthosporium-*Arten an Getreide,
- *Mycosphaerella-*Arten an Bananen und Erdnüssen,
- *Phytophthora infestans* an Kartoffeln und Tomaten,
- *Plasmopara viticola* an Reben,
- *Podosphaera leucotricha* an Äpfeln,
- Pseudocercosporella herpotrichoides an Weizen und Gerste,
- Pseudoperonospora-Arten an Hopfen und Gurken,
- *Puccinia-Arten* an Getreide,
- *Pyricularia* oryzae an Reis,
- Rhizoctonia-Arten an Baumwolle, Reis und Rasen,
- Septoria nodorum an Weizen,
- Uncinula necator an Reben,
- Ustilago-Arten an Getreide und Zuckerrohr, sowie
- Venturia-Arten (Schorf) an Äpfeln und Birnen.

Die Verbindungen I eignen sich außerdem zur Bekämpfung von Schadpilzen wie Paecilomyces variotii im Materialschutz (z.B. Holz, Papier, Dispersionen für den Anstrich, Fasern bzw. Gewebe) und im Vorratsschutz.

Die Verbindungen I werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung kann sowohl vor als auch nach der Infektion der Materialien, Pflanzen oder Samen durch die Pilze erfolgen.

Die fungiziden Mittel enthalten im allgemeinen zwischen 0,1 und 95, vorzugsweise zwischen 0,5 und 90 Gew.-% Wirkstoff.

Die Aufwandmengen liegen bei der Anwendung im Pflanzenschutz je nach Art des gewünschten Effektes zwischen 0,01 und 2,0 kg Wirkstoff pro ha.

Bei der Saatgutbehandlung werden im allgemeinen Wirkstoffmengen von 0,001 bis 0,1 g, vorzugsweise 0,01 bis 0,05 g je Kilogramm Saatgut benötigt.

Bei der Anwendung im Material- bzw. Vorratsschutz richtet sich die Aufwandmenge an Wirkstoff nach der Art des Einsatzgebietes und des gewünschten Effekts. Übliche Aufwandmengen sind im Materialschutz beispielsweise 0,001 g bis 2 kg, vorzugsweise 0,005 g bis 1 kg Wirkstoff pro Qubikmeter behandelten Materials.

Die Verbindungen I können in die üblichen Formulierungen überführt werden, z.B. Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung der erfindungsgemäßen Verbindung gewährleisten.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B.Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin-Sulfitablaugen und Methylcellulose.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate und Fettsäuren sowie deren Alkali- und Erdalkalisalze, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylcellulose in Betracht.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,01 und 95 Gew.-%, vorzugsweise zwischen 0,1 und 90 Gew.-% des Wirkstoffs. Die Wirkstoffe werden dabei in einer Reinheit von 90% bis 100%, vorzugsweise 95% bis 100% (nach NMR-Spektrum) eingesetzt.

Beispiele für Formulierungen sind:
I. 5 Gew.-Teile einer erfindungsgemäßen Verbindung werden mit 95 Gew.-Teilen feinteiligem Kaolin innig vermischt. Man erhält auf diese Weise ein Stäubemittel, das 5 Gew.-% des Wirkstoffs enthält.
II. 30 Gew.-Teile einer erfindungsgemäßen Verbindung werden mit einer Mischung aus 92 Gew.-Teilen pulverförmigem Kieselsäuregel und8 Gew.-Teilen Paraffinöl; das auf die Oberfläche dieses Kieselsäuregels gesprüht wurde, innig vermischt. Man erhält auf diese Weise eine Aufbereitung des Wirkstoffs mit guter Haftfähigkeit (Wirkstoffgehalt 23 Gew.-%).
III. 10 Gew.-Teile einer erfindungsgemäßen Verbindung werden in einer Mischung gelöst, die aus 90 Gew.-Teilen Xylol, 6 Gew.-Teilen des Anlagerungsproduktes von 8 bis 10 Mol Ethylenoxid an 1Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht (Wirkstoffgehalt 9 Gew.-%).
IV. 20 Gew.-Teile einer erfindungsgemäßen Verbindung werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5Gew.-Teilen des Anlagerungsproduktes von 40 Mol Ethylenoxid an 1 Mol Ricinusöl besteht (Wirkstoffgehalt 16 Gew.-%) .
V. 80 Gew.-Teile einer erfindungsgemäßen Verbindung werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen (Wirkstoffgehalt 80 Gew.-%).
VI. Man vermischt 90 Gew.-Teile einer erfindungsgemäßen Verbindung mit 10 Gew.-Teilen N-Methyl-α-pyrrolidon und erhält eine Lösung, die zur Anwendung in Form kleinster Tropfen geeignet ist (Wirkstoffgehalt 90 Gew.-%).
VII. 20 Gew.-Teile einer erfindungsgemäßen Verbindung werden in einer Mischung gelöst, die aus 40 Gew.-Teilen Cyclohexanon, 30Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.-% des Wirkstoffs enthält.
VIII. 20 Gew.-Teile einer erfindungsgemäßen Verbindung werden mit 3 Gew.-Teilen des Natriumsalzes der Diisobutylnaphthalin-α-sulfonsäure, 17 Gew.-Teilen des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20000 Gew.-Teilen Wasser erhält man eine Spritzbrühe, die 0,1 Gew.-% des Wirkstoffs enthält.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Die Wirkstoffkonzentrationen in den anwendungsfertigen Zubereitungen können in größeren Bereichen variiert werden. Im allgemeinen liegen sie zwischen 0,0001 und 10%, vorzugsweise zwischen 0,01 und 1%.

Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln. Beim Vermischen der Verbindungen I bzw. der sie enthaltenden Mittel in der Anwendungsform als Fungizide mit anderen Fungiziden erhält man in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
- Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zinkethylendiamin-bis-dithiocarbamat, Tetramethylthiuramdisulfide, Ammoniak-Komplex von Zink-(N,N-ethylen-bis-dithiocarbamat), Ammoniak-Komplex von Zink-(N,N'-propylen-bis-dithiocarbamat), Zink-(N,N'-propylenbis-dithiocarbamat), N,N'-Polypropylen-bis-(thiocarbamoyl)disulfid;
- Nitroderivate, wie Dinitro-(1-methylheptyl)-phenylcrotonat, 2-sec-Butyl-4, 6-dinitrophenyl-3,3-dimethylacrylat, 2-sec-Butyl-4,6-dinitrophenyl-isopropylcarbonat, 5-Nitro-isophthalsäure-di-isopropylester;
- heterocyclische Substanzen, wie 2-Heptadecyl-2-imidazolin-acetat, 2-Chlor-N-(4'-chlor-biphenyl-2-yl)-nicotinamid, 2,4-Dichlor-6-(o-chloranilino)-s-triazin, O,O-Diethyl-phthalimidophosphonothioat, 5-Amino-1-[bis-(dimethylamino)-phosphinyl]-3-phenyl-1,2,4- triazol, 2,3-Dicyano-1,4-dithioanthrachinon, 2-Thio-1,3-dithiolo[4,5-b]chinoxalin, 1-(Butylcarbamoyl)-2-benzimidazol-carbaminsäuremethylester, 2-Methoxycarbonylamino-benzimidazol, 2-(Furyl-(2))-benzimidazol, 2-(Thiazolyl-(4))-benzimidazol, N-(1,1,2,2-Tetrachlorethylthio)-tetrahydrophthalimid, N-Trichlormethylthio-tetrahydrophthalimid, N-Trichlormethylthio-phthalimid,
- N-Dichlorfluormethylthio-N',N'-dimethyl-N-phenyl-schwefelsäurediamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thio-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methylfuran-3-carbonsäureanilid, 2,5-Dimethyl-furan-3-carbonsäureanilid, 2,4,5-Trimethyl-furan-3-carbonsäureanilid, 2,5-Dimethyl-furan-3-carbonsäurecyclohexylamid, N-Cyclohexyl-N-methoxy-2,5-dimethyl-furan-3-carbonsäureamid, 2-Methyl-benzoesäure-anilid, 2-Iod-benzoesäure-anilid, N-Formyl-N-morpholin-2,2,2-trichlorethylacetal, Piperazin-1,4-diylbis-1-(2,2,2-trichlorethyl)-formamid, 1-(3,4-Dichloranilino)-1-formylamino-2,2,2-trichlorethan, 2,6-Dimethyl-N-tridecyl-morpholin bzw. dessen Salze, 2,6-Dimethyl-N-cyclododecyl-morpholin bzw. dessen Salze, N-[3-(p-tert.-Butylphenyl)-2-methylpropyl]-cis-2,6-dimethyl-morpholin, N-[3-(p-tert.-Butylphenyl)-2-methylpropyl]-piperidin, 1-[2-(2,4-Dichlorphenyl)-4-ethyl-1,3-dioxolan-2-yl-ethyl]-1H-1,2,4-triazol, 1-[2-(2,4-Dichlorphenyl)-4-n-propyl-1,3-dioxolan-2-yl-ethyl]-1H-1,2,4-triazol, N-(n-Propyl)-N-(2,4,6-trichlorphen-oxyethyl)-N'-imidazol-yl-harnstoff, 1-(4-Chlorphenoxy)-3,3-dimethyl-1-(1H-1,2,4-triazol-1-yl)-2-butanon, 1-(4-Chlorphen-oxy)-3,3-dimethyl-1-(1H-1,2,4-triazol-1-yl)-2-butanol, (2RS,3RS)-1-[3-(2-Chlorphenyl)-2-(4-fluorphenyl)-oxiran-2-ylmethyl]-1H-1,2,4-triazol, α-(2-Chlorphenyl)-α-(4-chlorphenyl)-5-pyrimidin-methanol, 5-Butyl-2-dimethylamino-4-hydroxy-6-methyl-pyrimidin, Bis-(p-chlorphenyl)-3-pyridinmethanol, 1,2-Bis-(3-ethoxycarbonyl-2-thioureido)-benzol, 1,2-Bis-(3-methoxycarbonyl-2-thioureido)-benzol,
- Strobilurine wie Methyl-E-methoxyimino-[α-(o-tolyloxy)-o-tolyl]acetat, Methyl-E-2-{2-[6-(2-cyanophenoxy)-pyrimidin-4-yloxy)-phenyl}-3-methoxyacrylat, Methyl-E-methoxyimino-[α-(2-phenoxyphenyl)]-acetamid, Methyl-E-methoxyimino-[α-(2,5-dimethylphenoxy)-o-tolyl]-acetamid, Methyl-E-2-{2-[2-trifluormethylpyridyl-6-]oxymethyl]-phenyl}3-methoxyacrylat, (E,E)-Methoximino-{2-[1-(3-trifluormethylphenyl)-ethylidenaminooxymethyl]-phenyl}-essigsäuremethylester, Methyl-N-(2-{[1-(4-chlorphenyl)-1H-pyrazol-3-yl]oxymethyl}phenyl)N-methoxy-carbamat,
- Anilinopyrimidine wie N-(4,6-Dimethylpyrimidin-2-yl)-anilin, N-[4-Methyl-6-(1-propinyl)-pyrimidin-2-yl]-anilin, N-[4-Methyl-6-cyclopropyl-pyrimidin-2-yl]-anilin,
- Phenylpyrrole wie 4-(2,2-Difluor-1,3-benzodioxol-4-yl)-pyrrol-3-carbonitril,
- Zimtsäureamide wie 3-(4-Chlorphenyl)-3-(3,4-dimethoxyphenyl)-acrylsäuremorpholid, 3-(4-Fluorphenyl)-3-(3,4-dimethoxyphenyl)-acrylsäuremorpholid,
- sowie verschiedene Fungizide, wie Dodecylguanidinacetat, 1-(3-Brom-6-methoxy-2-methyl-phenyl)-1-(2,3,4-trimethoxy-6-methyl-phenyl)-methanon, 3-[3-(3,5-Dimethyl-2-oxycyclohexyl)-2-hydroxyethyl]-glutarimid, Hexachlorbenzol, DL-Methyl-N-(2,6-dimethyl-phenyl)-N-furoyl(2)-alaninat, DL-N-(2,6-Dimethyl-phenyl)-N-(2'-methoxyacetyl)-alanin-methylester, N-(2,6-Dimethylphenyl)-N-chloracetyl-D,L-2-aminobutyrolacton, DL-N-(2,6-Dimethylphenyl)-N-(phenylacetyl)-alaninmethylester, 5-Methyl-5-vinyl-3-(3,5-dichlorphenyl)-2,4-di-oxo-1,3-oxazolidin, 3-(3,5-Dichlorphenyl)-5-methyl-5-methoxymethyl-1,3-oxazolidin- 2,4-dion, 3-(3,5-Dichlorphenyl)-1-isopropylcarbamoylhydantoin, N-(3,5-Dichlorphenyl)-1,2-dimethylcyclopropan-1,2-dicarbonsäureimid, 2-Cyano-[N-(ethylaminocarbonyl)-2-methoximino]-acetamid, 1-[2-(2,4-Dichlorphenyl)-pentyl]-1H-1,2,4-triazol, 2,4-Difluor-α-(1H-1,2,4-triazolyl-1-methyl)-benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)-methylsilyl)-methyl)-1H-1,2,4-triazol, 5-Chlor-2-cyano-4-p-tolyl-imidazol-1-sulfonsäuredimethylamid, 3,5-Dichlor-N-(3-chlor-1-ethyl-1-methyl-2-oxo-propyl)-4-methylbenzamid.

### Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in der anschließenden Tabelle mit physikalischen Angaben aufgeführt.

### Beispiel 1: Herstellung von Diethyl-(2-fluor-4-methylphenyl)-malonat

0,49 mol Diethylmalonat wurden innerhalb 2 Std. bei 60°C einer Mischung von 0,51 mol NaH und 140 ml 1,4-Dioxan zugestzt. Die Mischung wurde etwa 10 min bei 60°C gerührt, dann wurden 0,05 mol Cu(I)Br zugesetzt. Nach 15 min wurde eine Mischung von 0,25 mol 4-Brom-3-fluortolol und 10 ml 1,4-Dioxan zugegeben. Die Reaktionsmischung wurde etwa 15 Std. auf 100°C erhitzt, dann auf 15°C abgekühlt. Nach Ansäuern mit 35 ml 12N HCl bei 15 bis 20°C wurde der entstandene Niederschlag abfiltriert. Das Filtrat wurde extrahiert, die organischen Phasen nach Trennung getrocknet, dann vom Lösungsmittel befreit. Man erhielt 40 g der Titelverbindung.

### Beispiel 2: Herstellung von 5,7-Dihydroxy-6-(2-fluor-4-methylphenyl)-[1,2,4]-triazolo-[1,5-a]pyrimidin

Eine Mischung von 14 g 3-Amino-1,2,4-triazol, 0,17 mol Malonat aus Bsp. 1 und 50 ml Tributylamin wurde für 6 Std. auf 180°C erhitzt, dann auf 70°C abgekühlt. Nach Zusatz einer Lösung von 21 g NaOH in 200 ml Waser wurden weiter 30 min gerührt, dann die organische Phase abgetrennt und die wässrige Phase mit Diethlether extrahiert. Aus der wässrigen Phase wurde durch Ansäuern mit konz. HCl das Produkt ausgefällt. Nach Trocknung des abfiltrierten Niederschlages erhielt man 39 g der Titelverbindung.

### Beispiel 3: Herstellung von 5,7-Dichlor-6-(2-fluor-4-methylphenyl)-[1,2,4]-triazolo-[1,5-a]pyrimidin

Eine Mischung von 30 g der Verbindung aus Bsp. 2 und 50 ml POCl₃ wurde 8 Std. refluxiert, dabei destillierte ein Teil des POCl₃ ab. Der Rückstand wurde in eine Mischung von CH₂Cl₂ und Wasser gegeben, die organische Phase abgetrennt, dann getrocknet und vom Lösungsmittel befreit. Aus dem Rückstand erhielt man 26 g der Titelverbindung vom Fp. 152°C.

### Beispiel 4: Herstellung von 5-Chlor-6-(2-fluor-4-methylphenyl)-7-isopropylamino-[1,2,4]-triazolo[1,5-a]pyrimidin [I-2]

Eine Lösung von 1,5 mmol Isopropylamin und 1,5 mmol Triethylamin in 10 ml CH₂Cl₂ wurde unter Rühren zu einer Lösung von 1,5 mmol der Verbindung aus Bsp. 3 in 20 ml CH₂Cl₂ gegeben. Die Reaktionsmischung wurde 16 Std. bei 20 bis 25°C gerührt, dann mit verd. HCl gewaschen. Die organische Phase wurde abgetrennt, getrocknet und vom Lösungsmittel befreit. Aus dem Rückstand erhielt man nach Chromatographie an Kieselgel 420 mg der Tielverbindung vom Fp. 121°C .

### Beispiel 5: Herstellung von 5-Cyano-6-(2-fluor-4-methylphenyl)-7-(4-methylpiperidin-1-yl)-[1,2,4]-triazolo[1,5-a]pyrimidin

Eine Mischung von 0,1 mol der Verbindung I-3 und 0,25 mol N(C₂H₅)₄CN in 750 ml Dimethylformamid (DMF) wurde bei 20 - 25°C etwa 16 Std. gerührt. Nach Versetzen mit Wasser und Methyl-tert.Butylether (MTBE) wurde die organische Phase abgetrennt, mit Wasser gewaschen und nach Trocknen vom Lösungsmittel befreit. Nach Chromatogrphie an Kieselgel erhielt man aus dem Rückstand 6,23 g der Titelverbindung.

¹H-NMR: 8,5 (s); 7,3 (t); 7,2 (d); 7,05 (d); 3,95 (d); 3,65 (d); 2,8 (t); 2,8 (t) ; 2,5 (s); 1,6 (m); 1,5 (m); 1,3 (m); 1,0 (d).

### Beispiel 6: Herstellung von 5-Methoxy-6-(2-fluor-4-methylphenyl)-7-(4-methylpiperidin-1-yl)-[1,2,4]-triazolo[1,5-a]pyrimidin

Eine 30 Gew.-%ige Lösung von 71,5 mmol NaOCH₃ wurde mit einer Lösung von 65 mmol der Verbindung I-3 in 400 ml wasserfr. Methanol versetzt. Nach etwa 16 Std. Rühren bei 20 - 25°C wurde Methanol abdestilliert und der Rückstand in CH₂Cl₂ aufgenommen. Die organische Phase wurde mit Wasser gewaschen und nach Trocknen vom Lösungsmittel befreit. Aus dem Rückstand wurden nach Chromatographie an Kieselgel 3,98 g der Titelverbindung vom Fp. 201°C erhalten.

### Beispiel 7: Herstellung von 5-Methyl-6-(2-fluor-4-methylphenyl)-7-(4-methylpiperidin-1-yl)-[1,2,4]-triazolo[1,5-a]pyrimidin

Eine Mischung von 20 ml Diethylmalonat und 0,27 g (5,65 mmol) einer 50 Gew.-%igen Dispersion von NaH in Mineralöl in 50 ml Acetonitril wurde bei 20 - 25°C etwa zwei Std. gerührt. 4,71 mmol der Verbindung I-3 wurden zugesetzt, dann wurde die Mischung etwa 20 Std. bei 60°C gerührt. 50 ml wässr. NH₄Cl-Lösung wurden zugesetzt, dann wurde mit verd. HCl-Lösung angesäuert. Nach Extraktion mit MTBE und Phasentrennung wurden die organischen Phasen getrocknet und eingedampft. Der Rückstand wurde durch Chromatographie an Kieselgel gereinigt. Das Eluat wurde in konz. HCl-Lösung aufgenommen und 24 Std. bei 80°C gehalten. Nach Abkühlen wurde die Lösung mit wässr. NaOH-Lösung auf pH 5 eingestellt. Nach Extraktion mit MTBE und Phasentrennung wurden die organischen Phasen getrocknet und vom Lösungsmittel befreit. Aus dem Rückstand erhielt man nach Chromatographie an Kieselgel 0,72 g der Titelverbindung.

¹H-NMR: 8,3 (s); 7,05 (m); 3,75 (d); 3,45 (d); 2,7 (t); 2,7 (t); 2,5 (s); 2,35 (s); 1,6 (m); 1,5 (m); 1,3 (m); 0,9 (d).

**Tabelle I: Verbindungen der Formel I:**

| | | | | | | | |
|---|---|---|---|---|---|---|---|
| 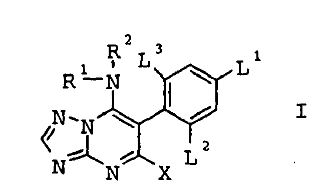 | | | | | | | |

| **Nr .** | **R**^{**1**} | **R**^{**2**} | **L**^{**1**} | **L**^{**2**} | **L**^{**3**} | **X** | **phys. Daten (Fp.[°C]).** |
|---|---|---|---|---|---|---|---|
| I-1 | CH₂C(CH₃)=CH₂ | CH₂CH₃ | CH₃ | F | H | Cl | 105 |
| I-2 | CH(CH₃)₂ | H | CH₃ | F | H | Cl | 121 |
| I-3 | -(CH₂)₂CH(CH₃)(CH₂)₂- | | CH₃ | F | H | Cl | 151 |
| I-4 | Cyclopentyl | H | CH₃ | F | H | Cl | 145 |
| I-5 | CH₂CH₃ | CH₂CH₃ | CH₃ | F | H | Cl | 127 |
| I-6 | CH₂CH₂CH₃ | CH₂CH₂CH₃ | CH₃ | F | H | Cl | 97 |
| I-7 | CH(CH₃)₂ | CH₃ | CH₃ | F | H | Cl | 188 |
| I-8 | (±) CH(CH₃)-CF₃ | H | CH₃ | F | H | Cl | A) 147; B) 139 |
| I-9 | (S) CH(CH₃)-CF₃ | H | CH₃ | F | H | Cl | A) 155; B) 154 |
| I-10 | (R) CH(CH₃)-CF₃ | H | CH₃ | F | H | Cl | A) 155; B) 154 |
| I-11 | CH₂CF₃ | H | CH₃ | F | H | Cl | 127 |
| I-12 | CH₂CF₂CF₃ | H | CH₃ | F | H | Cl | 187 |
| I-13 | CH₂CF₂CF₂CF₃ | H | CH₃ | F | H | Cl | 124 |
| I-14 | H | H | CH₃ | F | H | Cl | 247 |
| I-15 | -(CH₂)₂CH(CH₃)(CH₂)₂- | | CH₃ | F | H | CN | (s. Bsp. 5) |
| I-16 | -(CH₂)₂CH(CH₃) (CH₂)₂- | | CH₃ | F | H | OCH₃ | (s. Bsp. 6) |
| I-17 | -(CH₂)₂CH(CH₃)(CH₂)₂- | | CH₃ | F | H | CH₃ | (s. Bsp. 7) |
| I-18 | CH₂C(CH₃)=CH₂ | CH₂CH₃ | CH₃ | F | F | Cl | 123 |
| I-19 | -(CH₂)₂CH(CH₃) (CH₂)₂- | | CH₃ | F | F | Cl | 140 |
| I-20 | (±) CH(CH₃)-CF₃ | H | CH₃ | F | F | Cl | 154 |
| I-21 | (S) CH(CH₃)-CF₃ | H | CH₃ | F | F | Cl | 163 |
| I-22 | (R) CH(CH₃)-CF₃ | H | CH₃ | F | F | Cl | 163 |
| I-23 | CH₂CF₃ | H | CH₃ | F | F | Cl | 168 |

Im Fall von chiralen R¹-Gruppen können aufgrund der gehinderten Rotation der Phenylgruppe existieren zwei Diastereomeren A) und B), die sich in ihren physikalischen Eigenschaften unterscheiden können.

### Beispiele für die Wirkung gegen Schadpilze

Die fungizide Wirkung der Verbindungen der allgemeinen Formel I ließ sich durch die folgenden Versuche zeigen:

Die Wirkstoffe wurden getrennt oder gemeinsam als 10%ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Wettol® EM (nichtionischer Emulgator auf der Basis von ethoxyliertem Ricinusöl) aufbereitet und entsprechend der gewünschten Konzentration mit Wasser verdünnt.

Anwendungsbeispiel 1 - Wirksamkeit gegen die Dürrfleckenkrankheit der Tomate, verursacht durch *Alternaria solani*

Blätter von Topfpflanzen der Sorte "Große Fleischtomate St. Pierre" wurden mit einer wäßrigen Suspension, die aus einer Stammlösung bestehend aus 10 % Wirkstoff, 85 % Cyclohexanon und 5 % Emulgiermittel angesetzt wurde, bis zur Tropfnässe besprüht. Am folgenden Tag wurden die Blätter mit einer wäßrigen Zoosporenaufschwemmung von *Alternaria solani* in 2 % Biomalzlösung mit einer Dichte von 0,17 x 10⁶ Sporen/ml infiziert. Anschließend wurden die Pflanzen in einer wasserdampfgesättigten Kammer bei Temperaturen zwischen 20 und 22°C aufgestellt. Nach 5 Tagen hatte sich die Krautfäule auf den unbehandelten, jedoch infizierten Kontrollpflanzen so stark entwickelt, daß der Befall visuell in % ermittelt werden konnte.

In diesem Test zeigten die mit 250 ppm der Wirkstoffe Nr. I-1 bis 1-4, I-8A, I-8B, I-11 und 1-17 der Tabelle I behandelten Pflanzen nicht über 3 % Befall, während die unbehandelten Pflanzen zu 90 % befallen waren.

Anwendungsbeispiel 2 - Wirksamkeit gegen die Netzfleckenkrankheit der Gerste verursacht durch *Pyrenophora teres*

Blätter von in Töpfen gewachsenen Gerstenkeimlingen der Sorte "Igri" wurden mit wäßriger Wirkstoffaufbereitung, die aus einer Stammlösung bestehend aus 10 % Wirkstoff, 85 % Cyclohexanon und 5 % Emulgiermittel angesetzt wurde, bis zur Tropfnässe besprüht und 24 Stunden nach dem Antrocknen des Spritzbelages mit einer wäßrigen Sporensuspension von *Pyrenophora* [syn. *Drechslera*] *teres,* dem Erreger der Netzfleckenkrankheit inokuliert. Anschließend wurden die Versuchspflanzen im Gewächshaus bei Temperaturen zwischen 20 und 24°C und 95 bis 100 % relativer Luftfeuchtigkeit aufgestellt. Nach 6 Tagen wurde das Ausmaß der Krankheitsentwicklung visuell in % Befall der gesamten Blattfläche ermittelt.

In diesem Test zeigten die mit 250 ppm der Wirkstoffe Nr. 1-2 bis I-4, I-8B und I-11 der Tabelle I behandelten Pflanzen keinen bis maximal 7 % Befall, während die unbehandelten Pflanzen zu 100 % befallen waren.

Anwendungsbeispiel 3 - Protektive Wirksamkeit gegen Reisbrand verursacht durch *Pyricularia oryzae*

Blätter von in Töpfen gewachsenen Reiskeimlingen der Sorte "Tai-Nong 67" wurden mit wäßriger Wirkstoffaufbereitung, die mit einer Stammlösung bestehend aus 10 % Wirkstoff, 85 % Cyclohexanon und 5 % Emulgiermittel angesetzt wurde, bis zur Tropfnässe besprüht. Am folgenden Tag wurden die Pflanzen mit einer wäßrigen Sporensuspension von *Pyricularia oryzae* inokuliert. Anschließend wurden die Versuchspflanzen in Klimakammern bei 22 - 24°C und 95 - 99 % relativer Luftfeuchtigkeit für 6 Tage aufgestellt. Dann wurde das Ausmaß der Befallsentwicklung auf den Blättern visuell ermittelt.

In diesem Test zeigten die mit 250 ppm der Wirkstoffe Nr. I-1, I-3, I-4, I-8A, I-8B, I-11 und I-17 der Tabelle I behandelten Pflanzen nicht über 15 % Befall, während die unbehandelten Pflanzen zu 80 % befallen waren.

## Patentansprüche

1. Triazolopyrimidine der Formel I 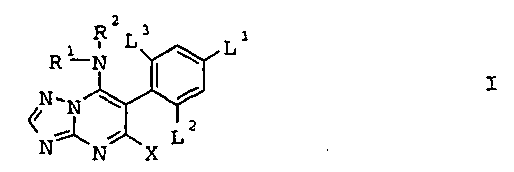 in die Substituenten folgende Bedeutung haben:
L¹ C₁-C₄-Alkyl;
L² Halogen;
L³ Wasserstoff oder Halogen;
X Halogen, Cyano, C₁-C₄-Alkyl, C₁-C₄-Alkoxy oder C₁-C₂-Halogenalkoxy;
R¹,R² unabhängig voneinander Wasserstoff, C₁-C₃-Alkyl, C₁-C₁₀-Halogenalkyl, C₃-C₈-Cycloalkyl, C₂-C₁₀-Alkenyl, C₄-C₁₀-Alkadienyl, C₂-C₁₀-Alkinyl oder
C₄-C₁₀-Alkyl, welches kein Chiralitätszentrum aufweist, oder
C₃-C₆-Cycloalkinyl, Phenyl, Naphthyl, oder ein fünf- bis zehngliedriger gesättigter, partiell ungesättigter oder aromatischer Heterocyclus, enthaltend ein bis vier Heteroatome aus der Gruppe O, N oder S,
R¹ und R² können auch zusammen mit dem Stickstoffatom, an das sie gebunden sind, einen fünf- oder sechs- gliedrigen Ring bilden, der durch ein Atom aus der Gruppe O, N und S unterbrochen sein und/oder einen oder mehrere Substituenten aus der Gruppe Halogen, C₁-C₆-Alkyl, C₁-C₆-Halogenalkyl und Oxy-C₁-C₃-alkylenoxy tragen kann oder in dem ein N- und ein benachbartes C-Atom durch eine C₁-C₄-Alkylenkette verbunden sein können;
wobei R¹ und/oder R² durch eine bis drei gleiche oder verschiedene Gruppen R^{a} substituiert sein kann:
R^{a} Halogen, Cyano, Nitro, Hydroxy, C₁-C₆-Alkyl, C₁-C₆-Halogenalkyl, C₁-C₆-Alkylcarbonyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₂-C₆-Alkenyl, C₂-C₆-Alkenyloxy, C₃-C₆-Alkinyloxy, C₃-C₆-Cycloalkyl, Phenyl, Naphthyl, fünf- bis zehngliedriger gesättigter, partiell ungesättigter oder aromatischer Heterocyclus, enthaltend ein bis vier Heteroatome aus der Gruppe O, N oder S,
wobei diese aliphatischen, alicyclischen oder aromatischen Gruppen ihrerseits partiell oder vollständig halogeniert sein oder eine bis drei Gruppen R^{b} tragen können:
R^{b} Halogen, Cyano, Nitro, Hydroxy, Mercapto, Amino, Carboxyl, Aminocarbonyl, Aminothiocarbonyl, Alkyl, Haloalkyl, Alkenyl, Alkenyloxy, Alkinyloxy, Alkoxy, Halogenalkoxy, Alkylthio, Alkylamino, Dialkylamino, Formyl, Alkylcarbonyl, Alkylsulfonyl, Alkylsulfoxyl, Alkoxycarbonyl, Alkylcarbonyloxy, Alkylaminocarbonyl, Dialkylaminocarbonyl, Alkylaminothiocarbonyl, Dialkylaminothiocarbonyl, wobei die Alkylgruppen in diesen Resten 1 bis 6 Kohlenstoffatome enthalten und die genannten Alkenyloder Alkinylgruppen in diesen Resten 2 bis 8 Kohlenstoffatome enthalten;
und/oder einen bis drei der folgenden Reste:
Cycloalkyl, Cycloalkoxy, Heterocyclyl, Heterocyclyloxy, wobei die cyclischen Systeme 3 bis 10 Ringglieder enthalten; Aryl, Aryloxy, Arylthio, Aryl-C₁-C₆-alkoxy, Aryl-C₁-C₆-alkyl, Hetaryl, Hetaryloxy, Hetarylthio, wobei die Arylreste vorzugsweise 6 bis 10 Ringglieder, die Hetarylreste 5 oder 6 Ringglieder enthalten, wobei die cyclischen Systeme partiell oder vollständig halogeniert oder durch Alkyloder Haloalkylgruppen substituiert sein können.

2. Verbindungen der Formel I gemäß Anspruch 1, in der R¹ und R² folgende Bedeutung haben:
R¹ ,R² unabhängig voneinander Wasserstoff, C₁-C₃-Alkyl, C₁-C₁₀-Halogenalkyl, C₃-C₈-Cycloalkyl, C₂-C₁₀-Alkenyl, C₄-C₁₀-Alkadienyl, C₂-C₁₀-Alkinyl oder
C₃-C₆-Cycloalkinyl, Phenyl, Naphthyl, oder ein fünfbis zehngliedriger gesättigter, partiell ungesättigter oder aromatischer Heterocyclus, enthaltend ein bis vier Heteroatome aus der Gruppe O, N oder S,
R¹ und R² können auch zusammen mit dem Stickstoffatom, an das sie gebunden sind, einen fünf- oder sechsgliedrigen Ring bilden, der durch ein Atom aus der Gruppe O, N und S unterbrochen sein und/oder einen oder mehrere Substituenten aus der Gruppe Halogen, C₁-C₆-Alkyl, C₁-C₆-Halogenalkyl und Oxy-C₁-C₃-alkylenoxy tragen kann oder in dem ein N- und ein benachbartes C-Atom durch eine C₁-C₄-Alkylenkette verbunden sein können;
wobei R¹ und/oder R² durch eine bis drei gleiche oder verschiedene Gruppen R^{a} substituiert sein kann.

3. Verfahren zur Herstellung der Verbindungen der Formel I gemäß Anspruch 1 in der X für Halogen, Cyano, C₁-C₄-Alkoxy oder C₁-C₂-Halogenalkoxy steht durch Umsetzung von 5-Aminotriazol der Formel II 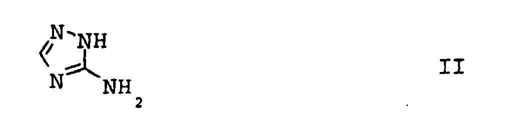 mit Phenylmalonaten der Formel III, 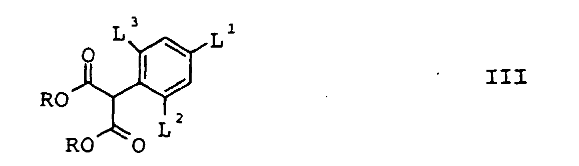 in der R für C₁-C₆-Alkyl steht, zu Dihydroxytriazolopyrimidinen der Formel IV 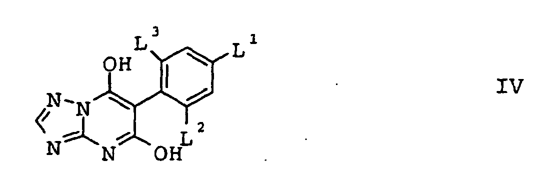 und Halogenierung zu den Dihalogenverbindungen der Formel V, 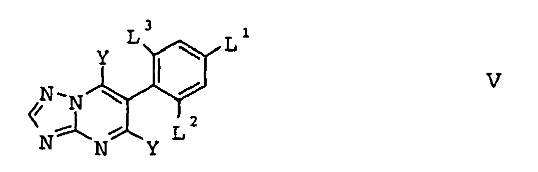 in der Y für Halogen, insbesondere Chlor oder Brom steht, Umsetzung mit Aminen der Formel VI, 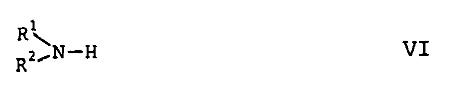 in der R¹ und R² die in Anspruch 1 gegebene Bedeutung haben, zu 5-Halogen-7-aminotriazolopyrimidinen der Formel I, in der X für Halogen steht, und, zur Herstellung von Verbindungen der Formel I, in der X Cyano, C₁-C₄-Alkoxy oder C₁-C₂-Halogenalkoxy bedeutet, Umsetzung mit Verbindungen der Formel VII,
M-X' VII
die, je nach Bedeutung der einzuführenden Gruppe X', ein anorganisches Cyanid, Alkoxylat oder Halogenalkoxylat darstellt und in der M für ein Ammonium-, Tetraalkylammonium-, Alkalimetall- oder Erdalkalimetallkation steht.

4. Verfahren zur Herstellung der Verbindungen der Formel I gemäß Anspruch 1, in der X für C₁-C₄-Alkyl steht, durch Umsetzung von 5-Aminotriazol der Formel II gemäß Anspruch 3 mit Dicarbonylverbindungen der Formel IIIa, 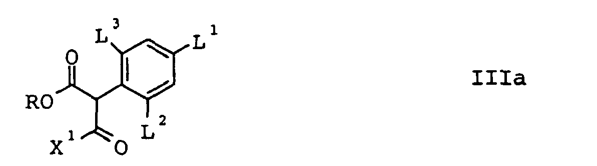 in der R und X¹ für C₁-C₄-Alkyl stehen zu Hydroxytriazolopyrimidinen der Formel IVa 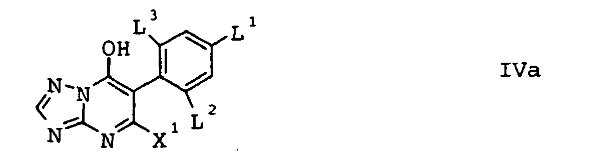 und Halogenierung zu Verbindungen der Formel V, 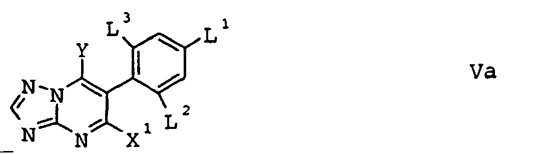 in der Y für Halogen, insbesondere Chlor oder Brom steht, Umsetzung mit Aminen der Formel VI gemäß Anspruch 3 zu Triazolopyrimidinen der Formel I, in der X für C₁-C₄-Alkyl steht.

5. Verbindungen der Formeln III, IIIa, IV und IVa gemäß Ansprüchen 3 und 4.

6. Zur Bekämpfung von Schadpilzen geeignetes Mittel, enthaltend einen festen oder flüssigen Trägerstoff und eine Verbindung der allgemeinen Formel I gemäß Anspruch 1.

7. Verwendung der Verbindungen I gemäß Anspruch 1 zur Herstellung eines zur Bekämpfung von Schadpilzen geeigneten Mittels.

8. Verfahren zur Bekämpfung von Schadpilzen, **dadurch gekennzeichnet, daß** man die Pilze oder die vor Pilzbefall zu schützenden Materialien, Pflanzen, den Boden oder Saatgüter mit einer wirksamen Menge einer Verbindung der allgemeinen Formel I gemäß Anspruch 1 behandelt.

## Claims

1. A triazolopyrimidine of the formula I 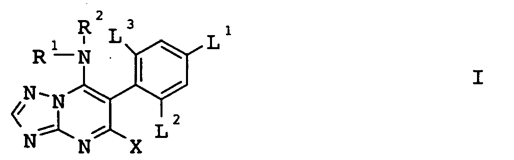 in which the substituents have the following meanings:
L¹ is C₁-C₄-alkyl;
L² is halogen;
L³ is hydrogen or halogen;
X is halogen, cyano, C₁-C₄-alkyl, C₁-C₄-alkoxy or C₁-C₂-haloalkoxy;
R¹,R² are, independently of one another, hydrogen, C₁-C₃-alkyl, C₁-C₁₀-haloalkyl, C₃-C₈-cycloalkyl, C₂-C₁₀-alkenyl, C₄-C₁₀-alkadienyl, C₂-C₁₀-alkynyl or C₄-C₁₀-alkyl, which has no center of chirality, or C₃-C₆-cycloalkynyl, phenyl, naphthyl, or a five- to ten-membered saturated, partially unsaturated or aromatic heterocycle, comprising one to four heteroatoms from the group consisting of O, N and S,
R¹ and R² can also, together with the nitrogen atom to which they are bonded, form a five- or six-membered ring which can be interrupted by an atom from the group consisting of O, N and S and/or can carry one or more
substituents from the group consisting of halogen, C₁-C₆-alkyl, C₁-C₆-haloalkyl and oxy-C₁-C₃-alkylenoxy or in which an N and a neighboring C atom can be connected via a C₁-C₄-alkylene chain;
wherein R¹ and/or R² can be substituted by one to three identical or different groups R^{a}:
R^{a} is halogen, cyano, nitro, hydroxyl, C₁-C₆-alkyl, C₁-C₆-haloalkyl, C₁-C₆-alkylcarbonyl, C₃-C₆-cycloalkyl, C₁-C₆-alkoxy, C₁-C₆-haloalkoxy, C₁-C₆-alkoxycarbonyl, C₁-C₆-alkylthio, C₁-C₆-alkylamino, di(C₁-C₆-alkyl)amino, C₂-C₆-alkenyl, C₂-C₆-alkenyloxy, C₃-C₆-alkynyloxy, C₃-C₆-cycloalkyl, phenyl, naphthyl or a five- to ten-membered saturated, partially unsaturated or aromatic heterocycle, comprising one to four heteroatoms from the group consisting of O, N and S,
wherein these aliphatic, alicyclic or aromatic groups, for their part, can be partially or completely halogenated or can carry one to three groups R^{b}:
R^{b} is halogen, cyano, nitro, hydroxyl, mercapto, amino, carboxyl, aminocarbonyl, aminothiocarbonyl, alkyl, haloalkyl, alkenyl, alkenyloxy, alkynyloxy, alkoxy, haloalkoxy, alkylthio, alkylamino, dialkylamino, formyl, alkylcarbonyl, alkylsulfonyl, alkylsulfoxyl, alkoxycarbonyl, alkylcarbonyloxy, alkylaminocarbonyl, dialkylaminocarbonyl, alkylaminothiocarbonyl or dialkylaminothiocarbonyl, wherein the alkyl groups in these radicals comprise 1 to 6 carbon atoms and the alkenyl or alkynyl groups mentioned in these radicals comprise 2 to 8 carbon atoms;
and/or one to three of the following radicals:
cycloalkyl, cycloalkoxy, heterocyclyl or heterocyclyloxy, wherein the cyclic systems comprise 3 to 10 ring members; aryl, aryloxy, arylthio, aryl-C₁-C₆-alkoxy, aryl-C₁-C₆-alkyl, hetaryl, hetaryloxy or hetarylthio, wherein
the aryl radicals preferably comprise 6 to 10 ring members and the hetaryl radicals comprise 5 or 6 ring members, wherein the cyclic systems can be partially or completely halogenated or can be substituted by alkyl or haloalkyl groups.

2. The compound of the formula I according to claim 1, in which R¹ and R² have the following meanings:
R¹,R² are, independently of one another, hydrogen, C₁-C₃-alkyl, C₁-C₁₀-haloalkyl, C₃-C₈-cycloalkyl, C₂-C₁₀-alkenyl, C₄-C₁₀-alkadienyl, C₂-C₁₀-alkynyl or C₃-C₆-cycloalkynyl, phenyl, naphthyl, or a five- to ten-membered saturated, partially unsaturated or aromatic heterocycle, comprising one to four heteroatoms from the group consisting of O, N and S,
R¹ and R² can also, together with the nitrogen atom to which they are bonded, form a five- or six-membered ring which can be interrupted by an atom from the group consisting of O, N and S and/or can carry one or more substituents from the group consisting of halogen, C₁-C₆-alkyl, C₁-C₆-haloalkyl and oxy-C₁-C₃-alkylenoxy or in which an N and a neighboring C atom can be connected via a C₁-C₄-alkylene chain;
wherein R¹ and/or R² can be substituted by one to three identical or different groups R^{a}.

3. A process for the preparation of the compound of the formula I according to claim 1 in which X is halogen, cyano, C₁-C₄-alkoxy or C₁-C₂-haloalkoxy, by reaction of 5-aminotriazole of the formula II 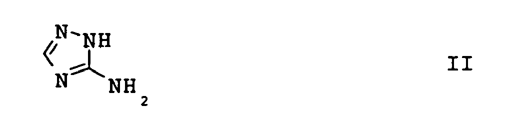 with phenylmalonates of the formula III, 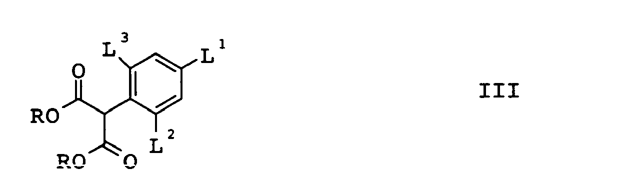 in which R is C₁-C₆-alkyl, to give dihydroxytriazolopyrimidines of the formula IV 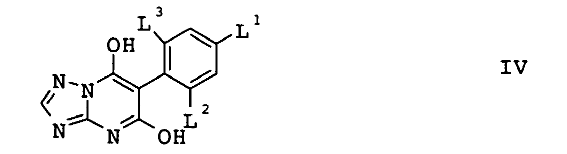 and halogenation to give the dihalogen compounds of the formula V, 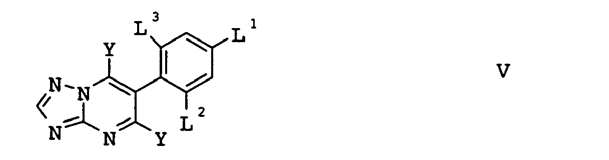 in which Y is halogen, in particular chlorine or bromine, reaction with amines of the formula VI, 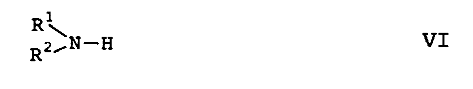 in which R¹ and R² have the meanings given in claim 1, to give 5-halo-7-aminotriazolopyrimidines of the formula I in which X is halogen, and, for the preparation of compounds of the formula I in which X represents cyano, C₁-C₄-alkoxy or C₁-C₂-haloalkoxy, reaction with compounds of the formula VII,
M-X' VII
which, according to the meaning of the group X' to be introduced, represents an inorganic cyanide, alkoxide or haloalkoxide and in which M is an ammonium, tetraalkylammonium, alkali metal or alkaline earth metal cation.

4. A process for the preparation of the compound of the formula I according to claim 1 in which X is C₁-C₄-alkyl, by reaction of 5-aminotriazole of the formula II according to claim 3 with dicarbonyl compounds of the formula IIIa, 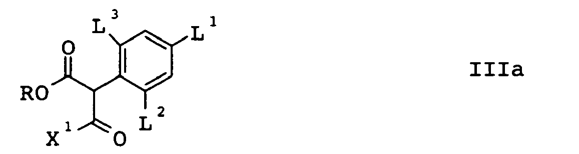 in which R and X¹ are C₁-C₄-alkyl, to give hydroxytriazolopyrimidines of the formula IVa 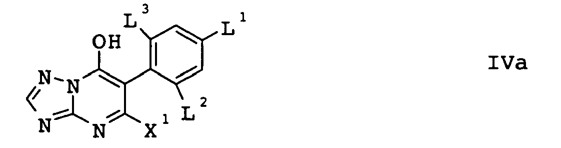 halogenation to give compounds of the formula Va, 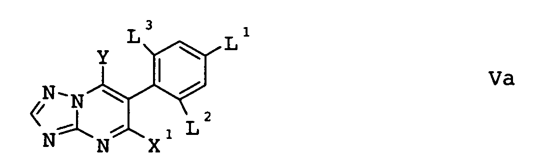 in which Y is halogen, in particular chlorine or bromine, and reaction with amines of the formula VI according to claim 3 to give triazolopyrimidines of the formula I in which X is C₁-C₄-alkyl.

5. A compound of the formulae III, IIIa, IV and IVa according to claim 3 or 4.

6. A composition suitable for the control of harmful fungi, comprising a solid or liquid carrier and a compound of the general formula I according to claim 1.

7. The use of the compound I according to claim 1 for the preparation of a composition suitable for the control of harmful fungi.

8. A method for the control of harmful fungi, which comprises treating the fungi or the materials, plants, ground or seeds to be protected from fungal attack with an effective amount of a compound of the general formula I according to claim 1.

## Revendications

1. Triazolopyrimidines de formul : 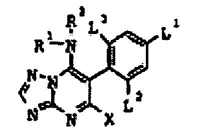 dans laquelle les substituants ont la signification suivante :
L¹ un radical C₁-C₄-alkyle;
L² un halogène;
L³ l'hydrogène ou un halogène;
X un halogène, un radical cyano, C₁-C₉-alkyle, C₁-C₉-alcoxy ou C₁-C₂-halogénalcoxy;
R¹, R² indépendamment l' un de l'autre, l'hydrogène, un radical C₁-C₃-alkyle, C₁-C₁₀₋halogénalkyle, C₃-C₈-cycloalkyle, C₂-C₁₀-alcényle, C₄-C₁₀-alcadiényle, C₂-C₁₀-alcinyle ou C₄-C₁₀-alkyle qui ne présente pas de centre de chiralité
ou
C₃-C₆-cycloalcinyle, phényle, naphthyle ou un hétérocycle saturé, partiellement insaturé ou aromatique penta- à décavalent contenant un à quatre hétéroatomes du groupe O, N ou S,
R¹ et R² peuvent également former avec l'atome d'azote auquel ils sont liés un cycle penta- ou hexavalent qui peut être interrompu par un atome du groupe O, N et S et/ou porter un ou plusieurs substituants du groupe halogène, C₁-C₆-alkyle, C₁₋C₆-halogénalkyle et oxy-C₁-C₃-akylèneoxy ou dans lequel un atome N et un atome C voisin peuvent être liés par une chaîne C₁-C₄-alkylène;
R¹ et/ou R² pouvant être substitués par un à trois groupes R^{a} identiques ou différents :
R^{a} un halogène, un groupe cyano, nitro, hydroxy, C₁-C₆-alkyle, C₁-C₆-halogénalkyle, C₁₋C₆-alkylcarbonyle, C₃-C₆-cycloalkyle, C₁-C₆₋alcoxy, C₁-C₆-halogénalcoxy, C₁-C₆₋alcoxycarbonyle, C₁-C₆-alkylthio, C₁-C₆₋alkylamino, di-C₁-C₆-alkylamino, C₂-C₆₋alcényle, C₂-C₆-alcényloxy, C₃-C₆-alcinyloxy, C₃-C₆-cycloalkyle, phényle, naphthyle, un hétérocycle saturé, partiellement insaturé ou aromatique penta- à décavalent contenant un à quatre hétéroatomes du groupe O, N ou S;
ces groupes aliphatiques, alicycliques ou aromatiques pouvant être pour leur part partiellement ou totalement halogénés ou porter un à trois groupes R^{b} :
R^{b} un halogène, un radical cyano, nitro, hydroxy, mercapto, amino, carboxyle, aminocarbonyle, aminothiocarbonyle, alkyle, haloalkyle, alcényle, alcényloxy, alcinyloxy, alcoxy, halogénalcoxy, alkylthio, alkylamino, dialkylamino, formyle, alkylcarbonyle, alkylsulfonyle, alkylsulfoxyle, alcoxycarbonyle, alkylcarbonyloxy, alkylaminocarbonyle, dialkylaminocarbonyle, alkylaminothiocarbonyle, dialkylaminothiocarbonyle, les groupes alkyle dans ces radicaux contenant 1 à 6 atomes de carbone et lesdits groupes alcényle ou alcinyle dans ces radicaux contenant 2 à 8 atomes de carbone;
et/ou un à trois des radicaux suivants :
cycloalkyle, cycloalcoxy, hétérocyclyle, hétérocyclyloxy, les systèmes cycliques étant tri- à décavalents; aryle, aryloxy, arylthio, aryl-C₁-C₆-alcoxy, aryl-C₁-C₆-alkyle, hétaryle, hétaryloxy, hétarylthio, les radicaux aryle étant de préférence hexa- à décavalents, les radicaux hétaryle penta- ou hexavalents, auquel cas les systèmes cycliques sont partiellement ou totalement halogénés ou peuvent être substitués par des groupes alkyle ou haloalkyle.

2. Composés de formule I selon la revendication 1 dans lesquels R¹ et R² ont la signification suivante :
R¹, R² indépendamment l'un de l'autre, l'hydrogène, un radical C₁-C₃-alkyle, C₁-C₁₀-halogénalkyle, C₃-C₈₋cycloalkyle, C₂-C₁₀-alcényle, C₄-C₁₀-alcadiényle, C₂-C₁₀-alcinyle ou
C₃-C₆-cycloalcinyle, phényle, naphthyle ou un hétérocycle saturé, partiellement insaturé ou aromatique penta- à décavalent contenant un à quatre hétéroatomes du groupe O, N ou S,
R¹ et R² peuvent également former avec l'atome d'azote auquel ils sont liés un cycle penta- ou hexavalent qui peut être interrompu par un atome du groupe O, N et S et/ou porter un ou plusieurs substituants du groupe halogène, C₁-C₆-alkyle, C₁₋C₆-halogénalkyle et oxy-C₁-C₃-akylèneoxy ou dans lequel un atome N et un atome C voisin peuvent être liés par une chaîne C₁-C₄-alkylène;
R¹ et/ou R² pouvant être substitués par un à trois groupes R^{a} identiques ou différents.

3. Procédé de préparation des composés de formule I selon la revendication 1 dans lequel X désigne un halogène, un radical cyano, C₁-C₄-alcoxy ou C₁-C₂₋halogénalcoxy par mise en réaction de 5-aminotriazole de formule II 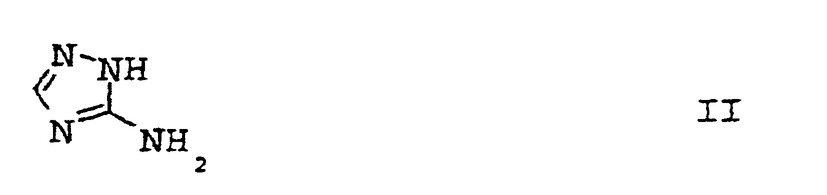 avec des phénylmalonates de formule III 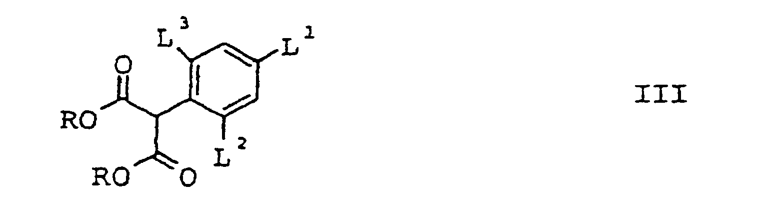 dans laquelle R désigne un radical C₁-C₆-alkyle en dihydroxytriazolopyrimidines de formule IV 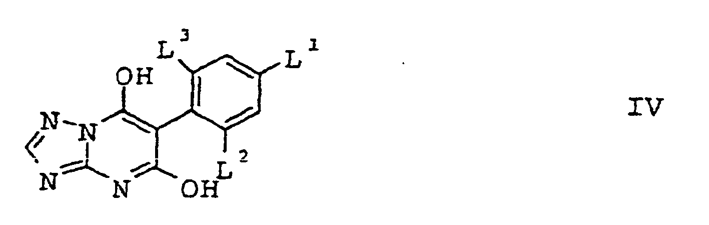 et halogénation en composés dihalogénés de formule V 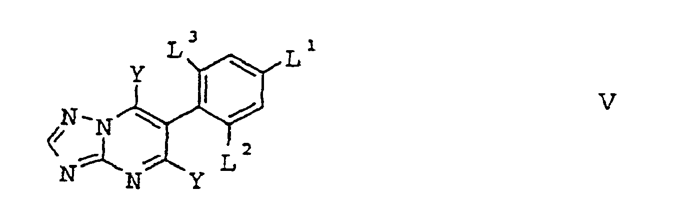 dans laquelle Y désigne un halogène, en particulier le chlore ou le brome, mise en réaction avec des amines de formule VI 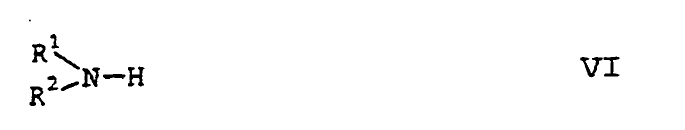 dans laquelle R¹ et R² ont la signification indiquée dans la revendication 1 en 5-halogéno-7-aminotriazolopyrimidines de formule I dans laquelle X désigne un halogène, et pour la préparation de composés de formule I dans laquelle X désigne un radical cyano, C₁-C₄-alcoxy ou C₁-C₂-halogénalcoxy, mise en réaction des composés de formule VII
M-X' VII
qui, en fonction de la signification du groupe d'introduction X', représentent un cyanure inorganique, un alcoxylate ou un halogénalcoxylate et dans laquelle M désigne un cation d'ammonium, de tétralkylammonium, de métal alcalin ou de métal alcalino-terreux.

4. Procédé de préparation des composés de formule I selon la revendication 1 dans lequel X désigne un radical C₁-C₄-alkyle, par mise en réaction de 5-aminotriazole de formule II selon la revendication 3 avec des composés dicarbonylés de formule IIIa 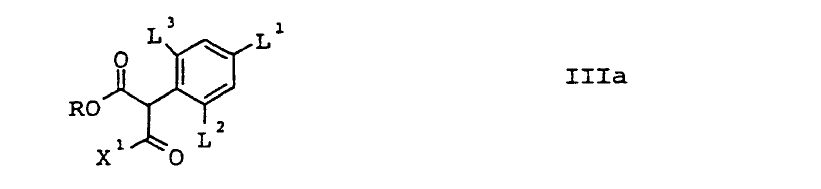 dans laquelle R et X¹ désignent un radical C₁-C₄-alkyle en hydroxytriazolopyrimidines de formule IVa 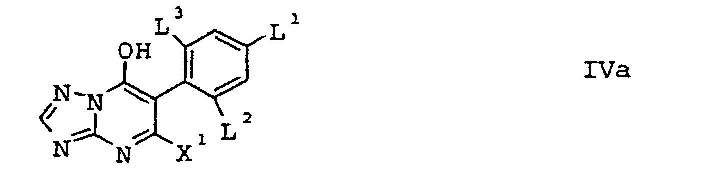 et halogénation en composés de formule Va 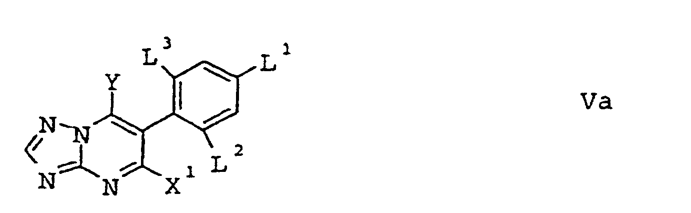 dans laquelle Y désigne un halogène, en particulier le chlore ou le brome, mise en réaction avec des amines de formule VI selon la revendication 3 en triazolopyrimidines de formule I dans laquelle X désigne un radical C₁-C₄-alkyle.

5. Composés de formules III, IIIa, IV et IVa selon les revendications 3 et 4.

6. Produit convenant pour la lutte contre les champignons nuisibles qui contient un excipient solide
ou liquide et un composé de formule générale I selon la revendication 1.

7. Utilisation des composés I selon la revendication 1 pour la préparation d'un produit convenant pour la lutte contre les champignons nuisibles.

8. Procédé de lutte contre les champignons nuisibles **caractérisé en ce que** l'on traite les champignons ou les matériaux, les plantes, le sol ou les semences à protéger contre une attaque fongique à l'aide d'une quantité active d'un composé de formule générale I selon la revendication 1.
